# EUROPEAN PATENT APPLICATION

(11) **EP 4 434 975 A1**
(43) Date of publication of application: **25.09.2024**
(21) Application number: 22896028.2
(22) Date of filing: 15.11.2022
(51) Int. Cl.: C07D 311/04, A61K 31/352, A61K 31/541, A61K 31/453, A61P 25/28, C07D 405/12, C07D 413/06, C07D 407/12, C07D 417/12

(54) **NOVEL CANNABICHROMENIC ACID DERIVATIVE, PREPARATION METHOD THEREFOR, AND COMPOSITION COMPRISING SAME FOR IMPROVING COGNITIVE FUNCTION**

(30) Priority: 16.11.2021 KR 20210157942
(71) Applicant: K-Medichem Co., Ltd., Gangwon-do 24232 (KR)
(72) Inventor: LEE, Koo Yeon, Chuncheon-si, Gangwon-do 24341 (KR); JUNG, Se-Hui, Chuncheon-si, Gangwon-do 24341 (KR); HEO, Hee-Young, Chuncheon-si, Gangwon-do 24277 (KR); KIM, Ju Eun, Namyangju-si, Gyeonggi-do 12168 (KR); LEE, Chan Bin, Chuncheon-si, Gangwon-do 24311 (KR)
(74) Representative: Graf von Stosch Patentanwaltsgesellschaft mbH
(86) International application number: PCT/KR2022/018008
(87) International publication number: WO 2023/090822

(57) **Abstract**

The present invention provides a cannabichromenic acid derivative, a method for preparing same, and a composition for cognitive function improvement comprising same. The novel cannabichromenic acid derivative of the present invention has been confirmed to exhibit acetylcholinesterase inhibitory activity and butyrylcholinesterase inhibitory activity, as well as inhibitory activity against monoamine oxidase B. Therefore, it is possible to simultaneously treat a decrease in acetylcholine levels in brain tissue and a decrease in blood butyrylcholine levels, which are seen in patients with dementia, and to provide a more effective cognitive function improvement treatment by preventing brain cell damage.

Therefore, the cannabichromenic acid derivative, the method for preparing same, and the composition for improving cognitive function comprising same, of the present invention, can be usefully used as a substance for improving cognitive function in the field of medicine and dietary supplement that can simultaneously inhibit acetylcholinesterase, butyrylcholinesterase, and monoamine oxidase B.

## Description

### FIELD

The present invention relates to novel cannabichromenic acid derivative, and more specifically, to novel cannabichromenic acid derivative, method for preparing same, and composition for cognitive function improvement comprising same.

### BACKGROUND

As we enter an aging society, the number of people living with dementia is increasing and the health and social costs are rising. As a result, dementia is increasingly becoming a national issue rather than an individual problem. Dementia is a pathological condition that should be distinguished from normal aging, and depending on the cause, it is classified into Alzheimer's disease, vascular dementia, other dementias such as alcoholism, trauma, and sequelae of Parkinson's disease, and more than 60% of dementia cases are known to be Alzheimer's disease. Dementia is characterized by a decline in intellectual functions such as memory, judgment, and language, a decline in daily living skills, and clinical features such as personality or behavioral disorders.

The pathogenesis of Alzheimer's disease is not clearly understood, but the toxicity of neurotoxic protein β-amyloid has been proposed as the most important cause. It is known to be produced by improper metabolism of amyloid precursor protein (APP), while normal metabolites of APP have been reported to be neuroprotective. Recent studies using genetic engineering methods have shown that toxic proteins such as β-amyloid accumulate in cells and blood vessels and are toxic to neurons, resulting in impaired brain functions.

To date, pharmacologic treatments for dementia have been based on increasing acetylcholine concentrations in the brain by administering acetylcholine precursors or drugs that inhibit the breakdown of acetylcholine. Typical drugs include tacrine, donepezil, rivastigmine, and galantamine.

Currently used dementia medications, such as the above, are acetylcholinesterase inhibitor-based compounds and they are known to have side effects in the digestive system, including nausea, vomiting, loss of appetite, and abdominal pain. In addition, as the activity of butyrylcholinesterase is dramatically increased in Alzheimer's disease, there is an urgent need to develop simultaneous inhibitors that not only inhibit acetylcholinesterase activity but also butyrylcholinesterase activity in order to treat Alzheimer's disease more effectively.

Meanwhile, monoamine oxidase (MAO) is an enzyme that catalyzes the oxidative deamination of neurotransmitters such as dopamine, serotonin, adrenaline, and noradrenaline, as well as non-biogenic amines, and there are two types of MAO-A and MAO-B. Neurodegenerative diseases such as Alzheimer's and Parkinson's are caused by oxidative stress caused by hydrogen peroxide from increased MAO activity, therefore MAO-B inhibitors can reduce oxygen radical formation and increase the amount of useful monoamines in the brain. Furthermore, in brain diseases and brain injury, including Alzheimer's disease, MAO-B promotes putrescine metabolism within reactive astrocytes, leading to the production of excess gamma-aminobutyric acid (GABA). Therefore, MAO-B inhibitors may act as inhibitors of GABA production by astrocytes to restore neural signaling and brain function, which may help improve symptoms of brain diseases and brain injury.

### OBJECT OF THE INVENTION

The problem to be addressed by the present invention is to provide a compound that simultaneously inhibits acetylcholinesterase activity and butyrylcholinesterase activity to more effectively improve cognitive dysfunction such as Alzheimer's dementia.

Further, the problem to be addressed by the present invention is to provide a compound that improves cognitive dysfunction such as degenerative brain disease by inhibiting MAO.

Furthermore, the problem to be addressed by the present invention is to provide a pharmaceutical composition or food composition comprising the compound showing the cognitive function improvement effect, which can be used for the purpose of improving cognitive function.

Furthermore, the problem to be addressed by the present invention is to provide a preparing method that can more efficiently synthesize the compound showing the cognitive function improvement effect.

The problem to be addressed by the present invention is not limited to the problems mentioned above, and other technical problems not mentioned can be clearly understood by those skilled in the art from the description below.

### SUMMARY

To address the problem above, according to one aspect of the present invention, there is provided a cannabichromenic acid derivative compound represented by the following Formula 1, a hydrate thereof, a solvate thereof or a pharmaceutically acceptable salt thereof, wherein: 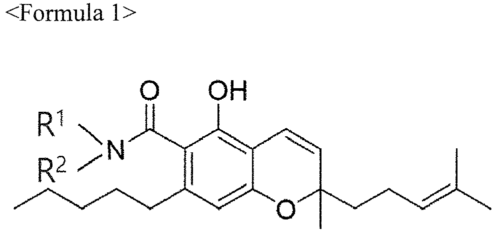
R¹ and R² are independently hydrogen; C₁₋₁₂ straight or branched alkyl chain; C₂₋₁₂ straight or branched alkenyl chain; C₂₋₅ straight or branched alkynyl chain; C₃₋₈ cycloalkyl; C₅₋₇ aryl; aryl C₁₋₄ alkyl; 5- to 7-membered heteroaryl having 1 to 3 heteroatom(s) selected from the group consisting of N and S; 5- to 7-membered heterocycloalkyl having 1 to 3 heteroatom(s) selected from the group consisting of N, O and S; or a fused bicyclic ring in which an aryl ring is fused with a non-aromatic cycloalkyl ring,
R¹ and R² are linked together to form a 5- to 7-membered non-aromatic heterocyclic ring having N linked to R¹ and R² and further having 0 to 2 heteroatoms selected from the group consisting of N and S,
the non-aromatic heterocyclic ring formed having R¹ and R² is fused with an aryl ring to form a fused bicyclic ring or not,
R¹ and R² are substituted with one or more R^{a} or not,
R^{a} is C₁₋₅ alkyl, halogen-substituted aryl, halogen-substituted heteroaryl, oxido, hydroxy, hydroxyalkyl, mercapto, alkylsulfonyl, halogen or butoxycarbonyl.

According to another aspect of the present invention, there is provided a pharmaceutical composition for cognitive function improvement, comprising the cannabichromenic acid derivative compound represented by the above Formula 1, a hydrate thereof, a solvate thereof or a pharmaceutically acceptable salt thereof as an active ingredient.

According to another aspect of the present invention, there is provided a pharmaceutical composition for preventing or treating cognitive function-related diseases, comprising the cannabichromenic acid derivative compound represented by the above Formula 1, a hydrate thereof, a solvate thereof or a pharmaceutically acceptable salt thereof as an active ingredient.

According to another aspect of the present invention, there is provided a method for preventing or treating cognitive function-related diseases, comprising: administering the cannabichromenic acid derivative compound represented by the above Formula 1, a hydrate thereof, a solvate thereof or a pharmaceutically acceptable salt thereof, to a subject in need thereof.

According to another aspect of the present invention, there is provided a use of the cannabichromenic acid derivative compound represented by the above Formula 1, a hydrate thereof, a solvate thereof or a pharmaceutically acceptable salt thereof, in manufacture of a medicament for preventing or treating cognitive function-related diseases.

According to another aspect of the present invention, there is provided a pharmaceutical composition for cognitive function improvement, characterized in that the cognitive function improvement is preventing or treating degenerative brain disease.

According to another aspect of the present invention, there is provided a composition for inhibiting monoamine oxidase (MAO) comprising the cannabichromenic acid derivative compound represented by the above Formula 1, a hydrate thereof, a solvate thereof or a pharmaceutically acceptable salt thereof as an active ingredient.

According to another aspect of the present invention, there is provided a food composition for cognitive function improvement comprising the cannabichromenic acid derivative compound represented by the above Formula 1, a hydrate thereof, a solvate thereof or a pharmaceutically acceptable salt thereof as an active ingredient.

According to another aspect of the present invention, there is provided a method for preparing a cannabichromenic acid derivative compound represented by the following Formula 1, comprising: reacting (E)-non-3-en-2-one represented by Formula 1-1 below with dimethyl malonate represented by Formula 1-2 below in an organic solvent to obtain a first compound represented by Formula 1a below; mixing the first compound obtained above with citral and calcium hydroxide, reacting in an organic solvent to obtain a second compound represented by Formula 2a below; reacting the second compound obtained above with thiophenol and cesium carbonate to obtain a third compound represented by Formula 3a; and reacting the third compound obtained above with 1-hydroxybenzotriazole, 1-(3-dimethylaminopropy)-3-ethylcarbodiimide hydrochloride and an amine compound, followed by purification to obtain a cannabichromenic acid derivative compound represented by Formula 1 below. 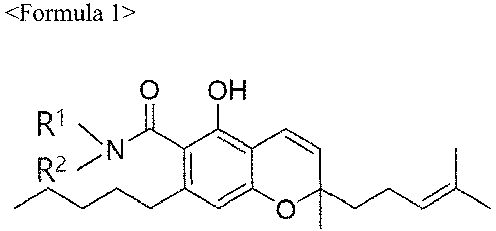 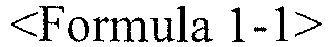 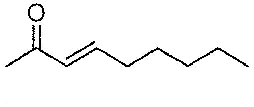 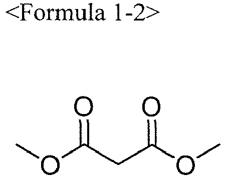 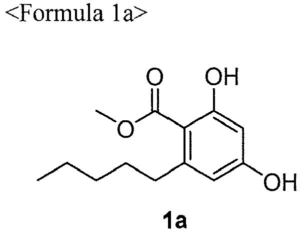 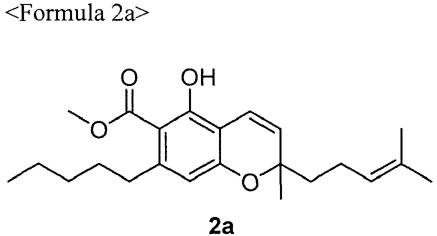 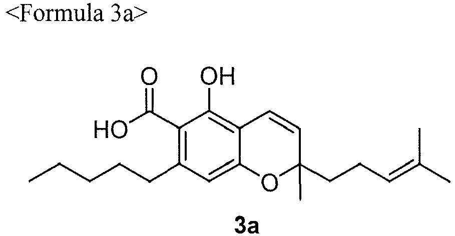 wherein in Formula 1, R¹ and R² are the same as defined in Claim 1.

The novel cannabichromenic acid derivatives of the present invention have been confirmed to exhibit acetylcholinesterase inhibitory and butyrylcholinesterase inhibitory effects, as well as inhibitory effects on monoamine oxidase B. It has been found that it is possible to simultaneously treat a decrease in the level of acetylcholine in the brain tissue and a decrease in the level of butyrylcholine in the blood, which are seen in patients with dementia, and to treat degenerative brain diseases more effectively by preventing brain cell damage, and that the amount of each drug can be reduced, thereby reducing side effects in the treatment of dementia.

Therefore, the cannabichromenic acid derivatives of the present invention, a method of preparing the same, and a composition for improving cognitive function comprising the same as an active ingredient can be usefully used as a substance for improving cognitive function in the field of medicine and dietary supplement that can simultaneously inhibit acetylcholinesterase, butyrylcholinesterase and monoamine oxidase B.

The effects of the present invention are not limited to the above effects, but should be understood to include all effects that can be inferred from the description of the invention or the composition of the invention as recited in the patent claims.

### BRIEF DESCRIPTION OF THE DRAWINGS

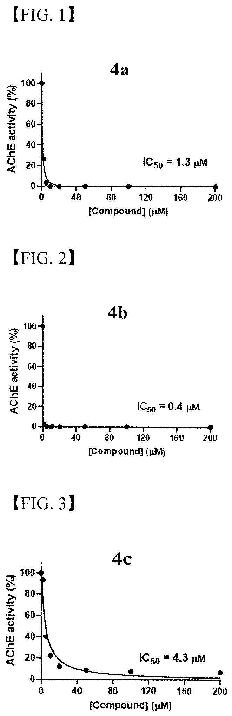

FIG. 1 is a graph showing the acetylcholinesterase (AChE) inhibitory efficacy (IC₅₀) of the synthesized cannabichromenic acid derivative (4a).
FIG. 2 is a graph showing the acetylcholinesterase (AChE) inhibitory efficacy (IC₅₀) of the synthesized cannabichromenic acid derivative (4b).
FIG. 3 is a graph showing the acetylcholinesterase (AChE) inhibitory efficacy (IC₅₀) of the synthesized cannabichromenic acid derivative (4c).

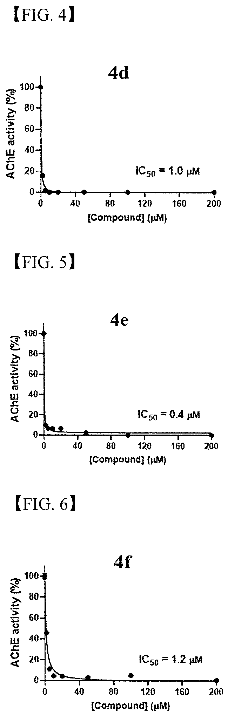

FIG. 4 is a graph showing the acetylcholinesterase (AChE) inhibitory efficacy (IC₅₀) of the synthesized cannabichromenic acid derivative (4d).
FIG. 5 is a graph showing the acetylcholinesterase (AChE) inhibitory efficacy (IC₅₀) of the synthesized cannabichromenic acid derivative (4e).
FIG. 6 is a graph showing the acetylcholinesterase (AChE) inhibitory efficacy (IC₅₀) of the synthesized cannabichromenic acid derivative (4f).

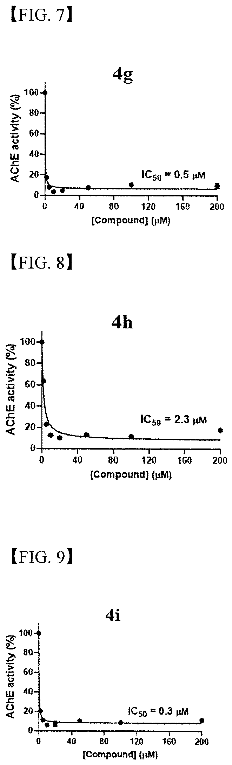

FIG. 7 is a graph showing the acetylcholinesterase (AChE) inhibitory efficacy (IC₅₀) of the synthesized cannabichromenic acid derivative (4g).
FIG. 8 is a graph showing the acetylcholinesterase (AChE) inhibitory efficacy (IC₅₀) of the synthesized cannabichromenic acid derivative (4h).
FIG. 9 is a graph showing the acetylcholinesterase (AChE) inhibitory efficacy (IC₅₀) of the synthesized cannabichromenic acid derivative (4i).

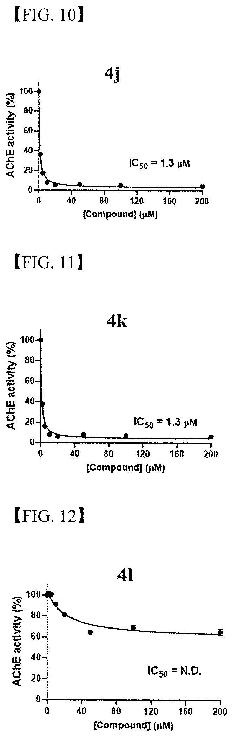

FIG. 10 is a graph showing the acetylcholinesterase (AChE) inhibitory efficacy (IC₅₀) of the synthesized cannabichromenic acid derivative (4j).
FIG. 11 is a graph showing the acetylcholinesterase (AChE) inhibitory efficacy (IC₅₀) of the synthesized cannabichromenic acid derivative (4k).
FIG. 12 is a graph showing the acetylcholinesterase (AChE) inhibitory efficacy (IC₅₀) of the synthesized cannabichromenic acid derivative (4l).

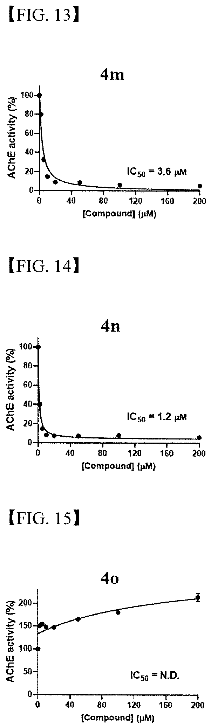

FIG. 13 is a graph showing the acetylcholinesterase (AChE) inhibitory efficacy (IC₅₀) of the synthesized cannabichromenic acid derivative (4m).
FIG. 14 is a graph showing the acetylcholinesterase (AChE) inhibitory efficacy (IC₅₀) of the synthesized cannabichromenic acid derivative (4n).
FIG. 15 is a graph showing the acetylcholinesterase (AChE) inhibitory efficacy (IC₅₀) of the synthesized cannabichromenic acid derivative (4o).

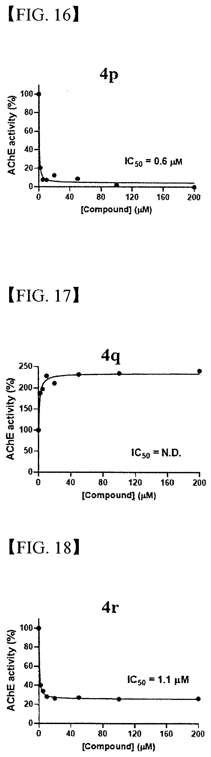

FIG. 16 is a graph showing the acetylcholinesterase (AChE) inhibitory efficacy (IC₅₀) of the synthesized cannabichromenic acid derivative (4p).
FIG. 17 is a graph showing the acetylcholinesterase (AChE) inhibitory efficacy (IC₅₀) of the synthesized cannabichromenic acid derivative (4q).
FIG. 18 is a graph showing the acetylcholinesterase (AChE) inhibitory efficacy (IC₅₀) of the synthesized cannabichromenic acid derivative (4r).

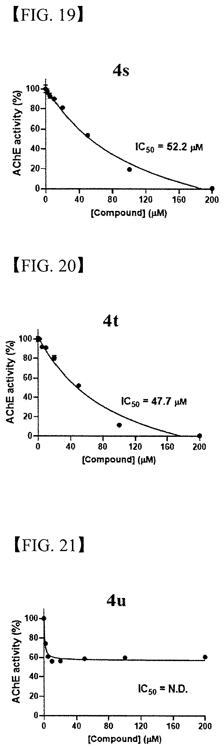

FIG. 19 is a graph showing the acetylcholinesterase (AChE) inhibitory efficacy (IC₅₀) of the synthesized cannabichromenic acid derivative (4s).
FIG. 20 is a graph showing the acetylcholinesterase (AChE) inhibitory efficacy (IC₅₀) of the synthesized cannabichromenic acid derivative (4t).
FIG. 21 is a graph showing the acetylcholinesterase (AChE) inhibitory efficacy (IC₅₀) of the synthesized cannabichromenic acid derivative (4u).

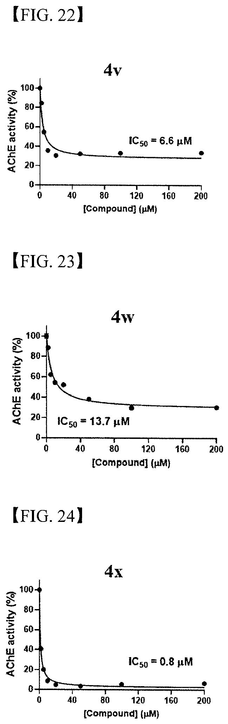

FIG. 22 is a graph showing the acetylcholinesterase (AChE) inhibitory efficacy (IC₅₀) of the synthesized cannabichromenic acid derivative (4v).
FIG. 23 is a graph showing the acetylcholinesterase (AChE) inhibitory efficacy (IC₅₀) of the synthesized cannabichromenic acid derivative (4w).
FIG. 24 is a graph showing the acetylcholinesterase (AChE) inhibitory efficacy (IC₅₀) of the synthesized cannabichromenic acid derivative (4x).

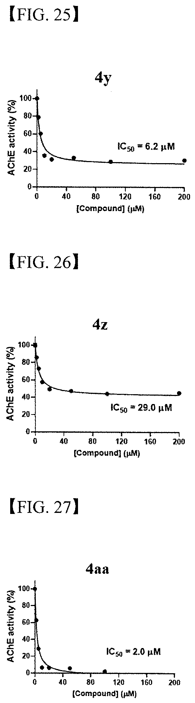

FIG. 25 is a graph showing the acetylcholinesterase (AChE) inhibitory efficacy (IC₅₀) of the synthesized cannabichromenic acid derivative (4y).
FIG. 26 is a graph showing the acetylcholinesterase (AChE) inhibitory efficacy (IC₅₀) of the synthesized cannabichromenic acid derivative (4z).
FIG. 27 is a graph showing the acetylcholinesterase (AChE) inhibitory efficacy (IC₅₀) of the synthesized cannabichromenic acid derivative (4aa).

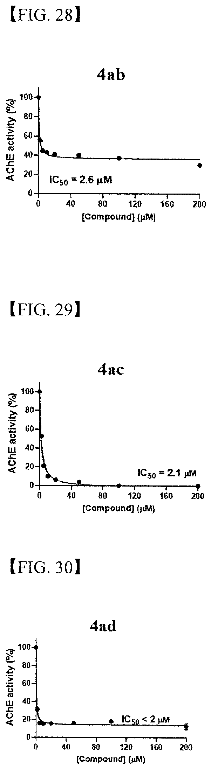

FIG. 28 is a graph showing the acetylcholinesterase (AChE) inhibitory efficacy (IC₅₀) of the synthesized cannabichromenic acid derivative (4ab).
FIG. 29 is a graph showing the acetylcholinesterase (AChE) inhibitory efficacy (IC₅₀) of the synthesized cannabichromenic acid derivative (4ac).
FIG. 30 is a graph showing the acetylcholinesterase (AChE) inhibitory efficacy (IC₅₀) of the synthesized cannabichromenic acid derivative (4ad).

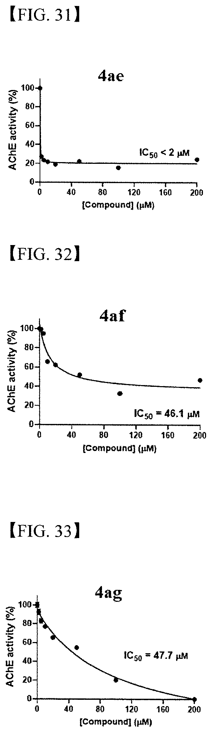

FIG. 31 is a graph showing the acetylcholinesterase (AChE) inhibitory efficacy (IC₅₀) of the synthesized cannabichromenic acid derivative (4ae).
FIG. 32 is a graph showing the acetylcholinesterase (AChE) inhibitory efficacy (IC₅₀) of the synthesized cannabichromenic acid derivative (4af).
FIG. 33 is a graph showing the acetylcholinesterase (AChE) inhibitory efficacy (IC₅₀) of the synthesized cannabichromenic acid derivative (4ag).

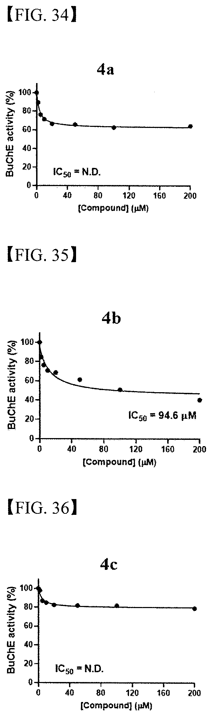

FIG. 34 is a graph showing the butyrylcholinesterase (BuChE) inhibitory efficacy (IC₅₀) of the synthesized cannabichromenic acid derivative (4a).
FIG. 35 is a graph showing the butyrylcholinesterase (BuChE) inhibitory efficacy (IC₅₀) of the synthesized cannabichromenic acid derivative (4b).
FIG. 36 is a graph showing the butyrylcholinesterase (BuChE) inhibitory efficacy (IC₅₀) of the synthesized cannabichromenic acid derivative (4c).

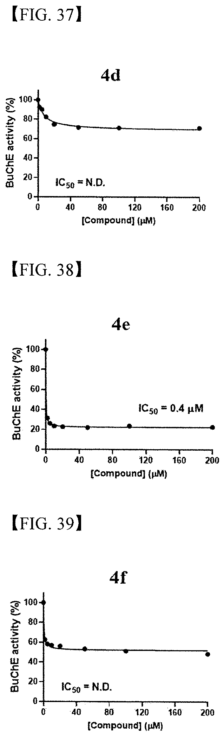

FIG. 37 is a graph showing the butyrylcholinesterase (BuChE) inhibitory efficacy (IC₅₀) of the synthesized cannabichromenic acid derivative (4d).
FIG. 38 is a graph showing the butyrylcholinesterase (BuChE) inhibitory efficacy (IC₅₀) of the synthesized cannabichromenic acid derivative (4e).
FIG. 39 is a graph showing the butyrylcholinesterase (BuChE) inhibitory efficacy (IC₅₀) of the synthesized cannabichromenic acid derivative (4f).

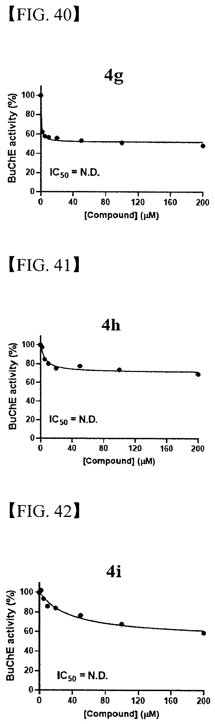

FIG. 40 is a graph showing the butyrylcholinesterase (BuChE) inhibitory efficacy (IC₅₀) of the synthesized cannabichromenic acid derivative (4g).
FIG. 41 is a graph showing the butyrylcholinesterase (BuChE) inhibitory efficacy (IC₅₀) of the synthesized cannabichromenic acid derivative (4h).
FIG. 42 is a graph showing the butyrylcholinesterase (BuChE) inhibitory efficacy (IC₅₀) of the synthesized cannabichromenic acid derivative (4i).

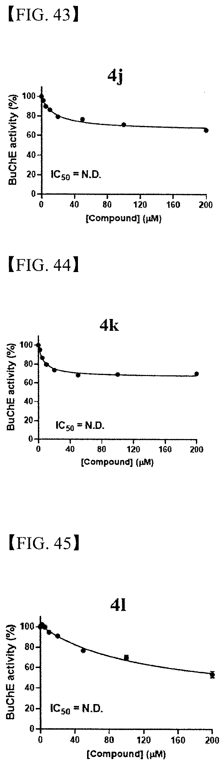

FIG. 43 is a graph showing the butyrylcholinesterase (BuChE) inhibitory efficacy (IC₅₀) of the synthesized cannabichromenic acid derivative (4j).
FIG. 44 is a graph showing the butyrylcholinesterase (BuChE) inhibitory efficacy (IC₅₀) of the synthesized cannabichromenic acid derivative (4k).
FIG. 45 is a graph showing the butyrylcholinesterase (BuChE) inhibitory efficacy (IC₅₀) of the synthesized cannabichromenic acid derivative (4l).

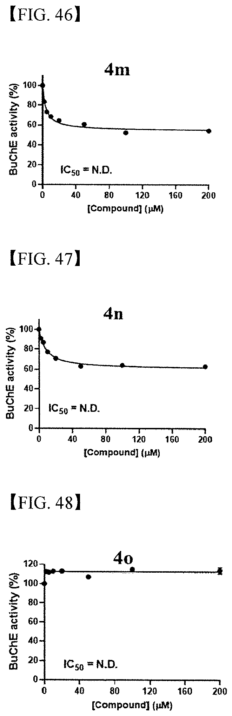

FIG. 46 is a graph showing the butyrylcholinesterase (BuChE) inhibitory efficacy (IC₅₀) of the synthesized cannabichromenic acid derivative (4m).
FIG. 47 is a graph showing the butyrylcholinesterase (BuChE) inhibitory efficacy (IC₅₀) of the synthesized cannabichromenic acid derivative (4n).
FIG. 48 is a graph showing the butyrylcholinesterase (BuChE) inhibitory efficacy (IC₅₀) of the synthesized cannabichromenic acid derivative (4o).

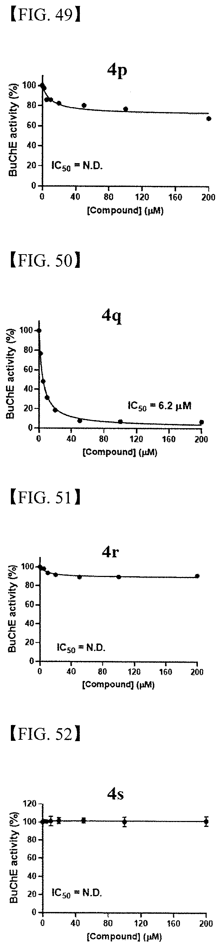

FIG. 49 is a graph showing the butyrylcholinesterase (BuChE) inhibitory efficacy (IC₅₀) of the synthesized cannabichromenic acid derivative (4p).
FIG. 50 is a graph showing the butyrylcholinesterase (BuChE) inhibitory efficacy (IC₅₀) of the synthesized cannabichromenic acid derivative (4q).
FIG. 51 is a graph showing the butyrylcholinesterase (BuChE) inhibitory efficacy (IC₅₀) of the synthesized cannabichromenic acid derivative (4r).
FIG. 52 is a graph showing the butyrylcholinesterase (BuChE) inhibitory efficacy (IC₅₀) of the synthesized cannabichromenic acid derivative (4s).

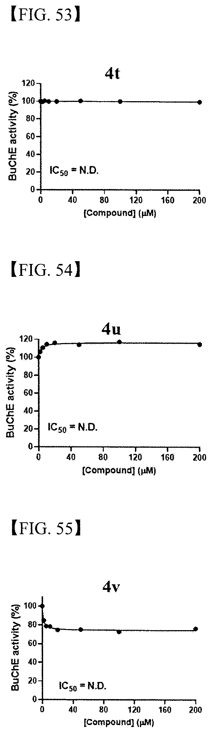

FIG. 53 is a graph showing the butyrylcholinesterase (BuChE) inhibitory efficacy (IC₅₀) of the synthesized cannabichromenic acid derivative (4t).
FIG. 54 is a graph showing the butyrylcholinesterase (BuChE) inhibitory efficacy (IC₅₀) of the synthesized cannabichromenic acid derivative (4u).
FIG. 55 is a graph showing the butyrylcholinesterase (BuChE) inhibitory efficacy (IC₅₀) of the synthesized cannabichromenic acid derivative (4v).

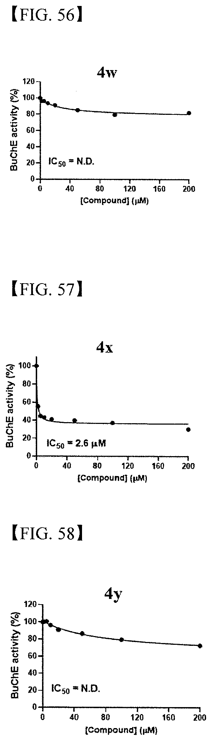

FIG. 56 is a graph showing the butyrylcholinesterase (BuChE) inhibitory efficacy (IC₅₀) of the synthesized cannabichromenic acid derivative (4w).
FIG. 57 is a graph showing the butyrylcholinesterase (BuChE) inhibitory efficacy (IC₅₀) of the synthesized cannabichromenic acid derivative (4x).
FIG. 58 is a graph showing the butyrylcholinesterase (BuChE) inhibitory efficacy (IC₅₀) of the synthesized cannabichromenic acid derivative (4y).

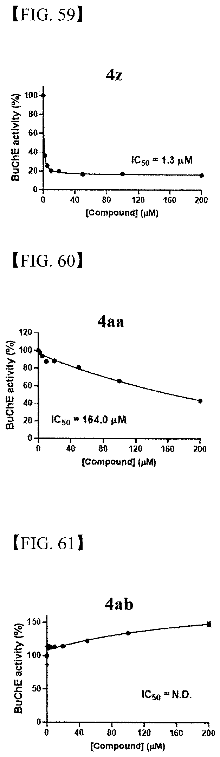

FIG. 59 is a graph showing the butyrylcholinesterase (BuChE) inhibitory efficacy (IC₅₀) of the synthesized cannabichromenic acid derivative (4z).
FIG. 60 is a graph showing the butyrylcholinesterase (BuChE) inhibitory efficacy (IC₅₀) of the synthesized cannabichromenic acid derivative (4aa).
FIG. 61 is a graph showing the butyrylcholinesterase (BuChE) inhibitory efficacy (IC₅₀) of the synthesized cannabichromenic acid derivative (4ab).

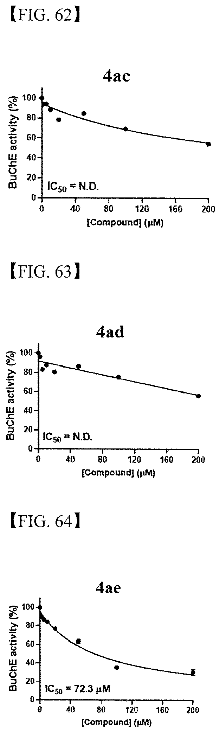

FIG. 62 is a graph showing the butyrylcholinesterase (BuChE) inhibitory efficacy (IC₅₀) of the synthesized cannabichromenic acid derivative (4ac).
FIG. 63 is a graph showing the butyrylcholinesterase (BuChE) inhibitory efficacy (IC₅₀) of the synthesized cannabichromenic acid derivative (4ad).
FIG. 64 is a graph showing the butyrylcholinesterase (BuChE) inhibitory efficacy (IC₅₀) of the synthesized cannabichromenic acid derivative (4ae).

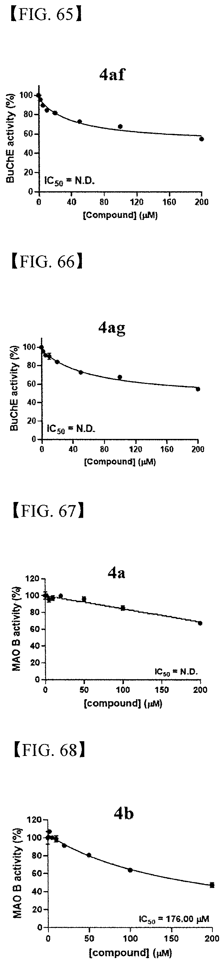

FIG. 65 is a graph showing the butyrylcholinesterase (BuChE) inhibitory efficacy (IC₅₀) of the synthesized cannabichromenic acid derivative (4af).
FIG. 66 is a graph showing the butyrylcholinesterase (BuChE) inhibitory efficacy (IC₅₀) of the synthesized cannabichromenic acid derivative (4ag).
FIG. 67 is a graph showing the monoamine oxidase (MAO) inhibitory efficacy (IC₅₀) of the synthesized cannabichromenic acid derivative (4a).
FIG. 68 is a graph showing the monoamine oxidase (MAO) inhibitory efficacy (IC₅₀) of the synthesized cannabichromenic acid derivative (4b).

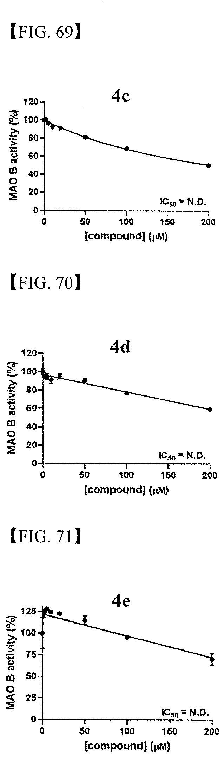

FIG. 69 is a graph showing the monoamine oxidase (MAO) inhibitory efficacy (IC₅₀) of the synthesized cannabichromenic acid derivative (4c).
FIG. 70 is a graph showing the monoamine oxidase (MAO) inhibitory efficacy (IC₅₀) of the synthesized cannabichromenic acid derivative (4d).
FIG. 71 is a graph showing the monoamine oxidase (MAO) inhibitory efficacy (IC₅₀) of the synthesized cannabichromenic acid derivative (4e).

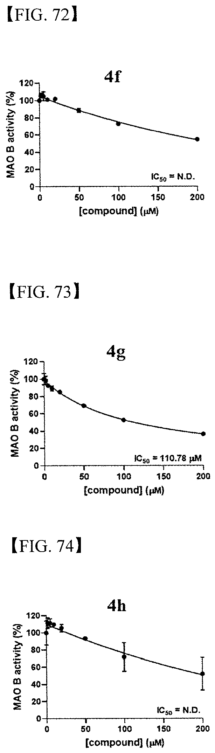

FIG. 72 is a graph showing the monoamine oxidase (MAO) inhibitory efficacy (IC₅₀) of the synthesized cannabichromenic acid derivative (4f).
FIG. 73 is a graph showing the monoamine oxidase (MAO) inhibitory efficacy (IC₅₀) of the synthesized cannabichromenic acid derivative (4g).
FIG. 74 is a graph showing the monoamine oxidase (MAO) inhibitory efficacy (IC₅₀) of the synthesized cannabichromenic acid derivative (4h).

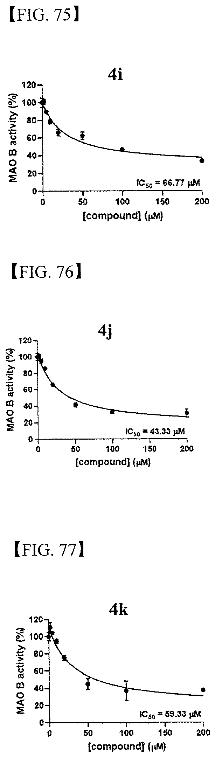

FIG. 75 is a graph showing the monoamine oxidase (MAO) inhibitory efficacy (IC₅₀) of the synthesized cannabichromenic acid derivative (4i).
FIG. 76 is a graph showing the monoamine oxidase (MAO) inhibitory efficacy (IC₅₀) of the synthesized cannabichromenic acid derivative (4j).
FIG. 77 is a graph showing the monoamine oxidase (MAO) inhibitory efficacy (IC₅₀) of the synthesized cannabichromenic acid derivative (4k).

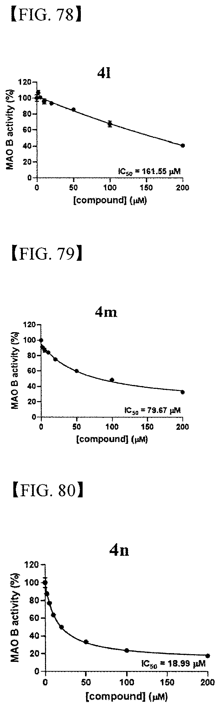

FIG. 78 is a graph showing the monoamine oxidase (MAO) inhibitory efficacy (IC₅₀) of the synthesized cannabichromenic acid derivative (4l).
FIG. 79 is a graph showing the monoamine oxidase (MAO) inhibitory efficacy (IC₅₀) of the synthesized cannabichromenic acid derivative (4m).
FIG. 80 is a graph showing the monoamine oxidase (MAO) inhibitory efficacy (IC₅₀) of the synthesized cannabichromenic acid derivative (4n).

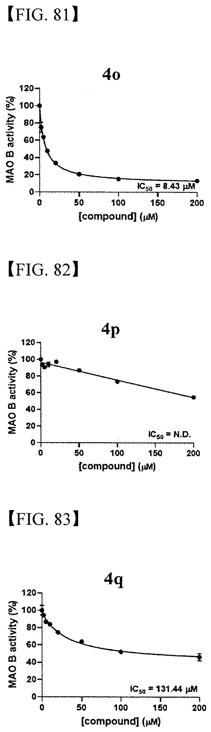

FIG. 81 is a graph showing the monoamine oxidase (MAO) inhibitory efficacy (IC₅₀) of the synthesized cannabichromenic acid derivative (4o).
FIG. 82 is a graph showing the monoamine oxidase (MAO) inhibitory efficacy (IC₅₀) of the synthesized cannabichromenic acid derivative (4p).
FIG. 83 is a graph showing the monoamine oxidase (MAO) inhibitory efficacy (IC₅₀) of the synthesized cannabichromenic acid derivative (4q).

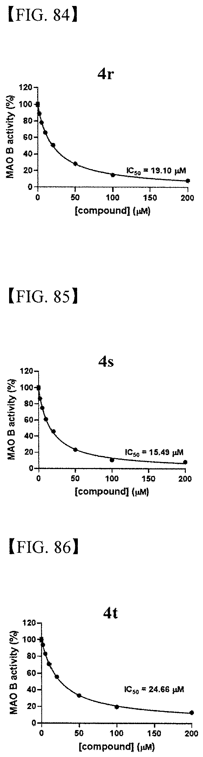

FIG. 84 is a graph showing the monoamine oxidase (MAO) inhibitory efficacy (IC₅₀) of the synthesized cannabichromenic acid derivative (4r).
FIG. 85 is a graph showing the monoamine oxidase (MAO) inhibitory efficacy (IC₅₀) of the synthesized cannabichromenic acid derivative (4s).
FIG. 86 is a graph showing the monoamine oxidase (MAO) inhibitory efficacy (IC₅₀) of the synthesized cannabichromenic acid derivative (4t).

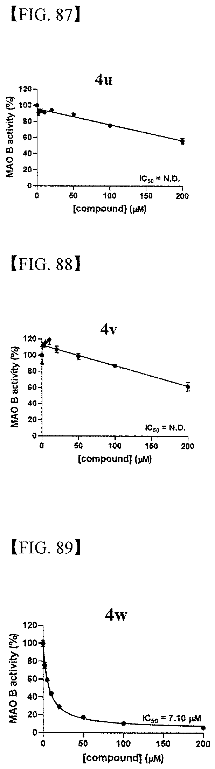

FIG. 87 is a graph showing the monoamine oxidase (MAO) inhibitory efficacy (IC₅₀) of the synthesized cannabichromenic acid derivative (4u).
FIG. 88 is a graph showing the monoamine oxidase (MAO) inhibitory efficacy (IC₅₀) of the synthesized cannabichromenic acid derivative (4v).
FIG. 89 is a graph showing the monoamine oxidase (MAO) inhibitory efficacy (IC₅₀) of the synthesized cannabichromenic acid derivative (4w).

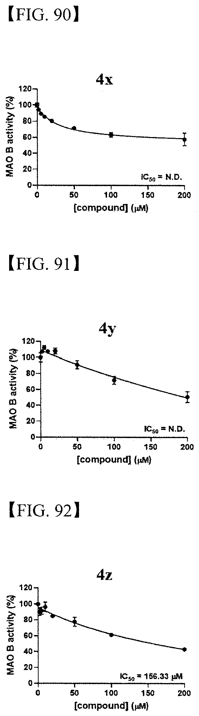

FIG. 90 is a graph showing the monoamine oxidase (MAO) inhibitory efficacy (IC₅₀) of the synthesized cannabichromenic acid derivative (4x).
FIG. 91 is a graph showing the monoamine oxidase (MAO) inhibitory efficacy (IC₅₀) of the synthesized cannabichromenic acid derivative (4y).
FIG. 92 is a graph showing the monoamine oxidase (MAO) inhibitory efficacy (IC₅₀) of the synthesized cannabichromenic acid derivative (4z).

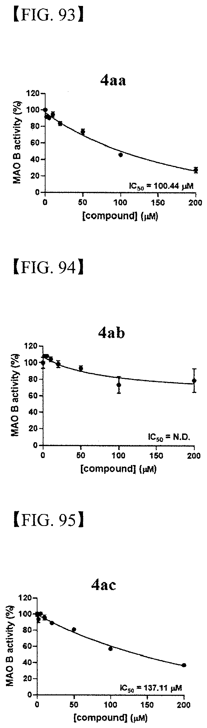

FIG. 93 is a graph showing the monoamine oxidase (MAO) inhibitory efficacy (IC₅₀) of the synthesized cannabichromenic acid derivative (4aa).
FIG. 94 is a graph showing the monoamine oxidase (MAO) inhibitory efficacy (IC₅₀) of the synthesized cannabichromenic acid derivative (4ab).
FIG. 95 is a graph showing the monoamine oxidase (MAO) inhibitory efficacy (IC₅₀) of the synthesized cannabichromenic acid derivative (4ac).

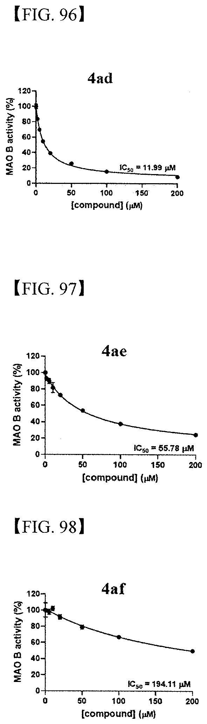

FIG. 96 is a graph showing the monoamine oxidase (MAO) inhibitory efficacy (IC₅₀) of the synthesized cannabichromenic acid derivative (4ad).
FIG. 97 is a graph showing the monoamine oxidase (MAO) inhibitory efficacy (IC₅₀) of the synthesized cannabichromenic acid derivative (4ae).
FIG. 98 is a graph showing the monoamine oxidase (MAO) inhibitory efficacy (IC₅₀) of the synthesized cannabichromenic acid derivative (4af).

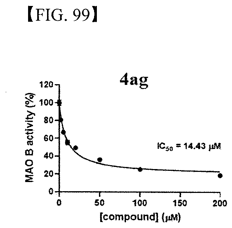

FIG. 99 is a graph showing the monoamine oxidase (MAO) inhibitory efficacy (IC₅₀) of the synthesized cannabichromenic acid derivative (4ag).

### DETAILED DESCRIPTION OF THE INVENTION

The present invention provides a cannabichromenic acid derivative compound represented by the following Formula 1, a hydrate thereof, a solvate thereof or a pharmaceutically acceptable salt thereof, wherein: 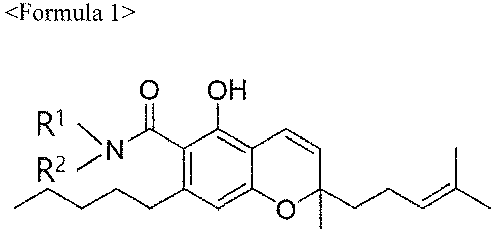
R¹ and R² are independently hydrogen; C₁₋₁₂ straight or branched alkyl chain; C₂₋₁₂ straight or branched alkenyl chain; C₂₋₅ straight or branched alkynyl chain; C₃₋₈ cycloalkyl; C₅₋₇ aryl; aryl C₁₋₄ alkyl; 5- to 7-membered heteroaryl having 1 to 3 heteroatom(s) selected from the group consisting of N and S; 5- to 7-membered heterocycloalkyl having 1 to 3 heteroatom(s) selected from the group consisting of N, O and S; or a fused bicyclic ring in which an aryl ring is fused with a non-aromatic cycloalkyl ring,
R¹ and R² are linked together to form a 5- to 7-membered non-aromatic heterocyclic ring having N linked to R¹ and R² and further having 0 to 2 heteroatoms selected from the group consisting of N and S,
the non-aromatic heterocyclic ring formed having R¹ and R² is fused with an aryl ring to form a fused bicyclic ring or not,
R¹ and R² are substituted with one or more R^{a} or not,
R^{a} is C₁₋₅ alkyl, halogen-substituted aryl, halogen-substituted heteroaryl, oxido, hydroxy, hydroxyalkyl, mercapto, alkylsulfonyl, halogen or butoxycarbonyl.

In one embodiment, R¹ and R² may be independently methyl, ethyl, isopropyl, butyl, nonyl, dodecyl, hexyl, undecenyl, octadienyl, propynyl, cyclopropyl, cyclopentyl, cyclohexyl, cycloheptyl, phenyl, pentyl, phenylpropyl, thiazolyl, tetrahydropyranyl, piperidinyl, or dihydroindenyl, or R¹ and R² are linked together to form thiomorpholino, piperazinyl, or dihydroisoquinolinyl.

In one embodiment, R^{a} may be methyl, ethyl, fluorophenyl, bromothiophenyl, oxido, hydroxy, hydroxyethyl, mercapto, methylsulfonyl, bromo or butoxycarbonyl.

Representative examples of cannabichromenic acid derivative compounds according to the present invention are as follows:
[1] 5-hydroxy-*N*-isopropyl-2-methyl-2-(4-methylpent-3-en-1-yl)-7-pentyl-*2H-*chromen-6-carboxamide (4a),
[2] 5-hydroxy-2-methyl-2-(4-methylpent-3-en-1-yl)-7-pentyl-*N*-phenyl-*2H* -chromen-6- carboxamide (4b),
[3] *N*-cyclopropyl-5-hydroxy-2-methyl-2-(4-methylpent-3-en-1-yl)-7-pentyl-*2H-*chromen-6-carboxamide (4c),
[4] *N*-butyl-5-hydroxy-2-methyl-2-(4-methylpent-3-en-1-yl)-7-pentyl-*2H*-chromen-6-carboxamide (4d),
[5] *N*-cyclopentyl-5-hydroxy-2-methyl-2-(4-methylpent-3-en-1-yl)-7-pentyl-*2H-*chromen-6-carboxamide (4e),
[6] *N*-cyclohexyl-5-hydroxy-2-methyl-2-(4-methylpent-3-en-1-yl)-7-pentyl-*2H-*chromen-6-carboxamide (4f),
[7] 5-hydroxy-2-methyl-2-(4-methylpent-3-en-1-yl)-*N*-nonyl-7-pentyl-*2H*-chromen-6-carboxamide (4g),
[8] *N*-dodecyl-5-hydroxy-2-methyl-2-(4-methylpent-3-en-1-yl)-7-pentyl-*2H-*chromen-6-carboxamide (4h),
[9] 5-hydroxy-2-methyl-2-(4-methylpent-3-en-1-yl)-7-pentyl-*N*- (undec-10-en-1-yl)-*2H*-chromen-6-carboxamide (4i),
[10] *N*-(2-ethylhexyl)-5-hydroxy-2-methyl-2-(4-methylpent-3-en-1-yl)-7-pentyl-*2H-*chromen-6-carboxamide (4j),
[11] (E)-*N*-(3,7-dimethylocta-2,6-dien-1-yl)-5-hydroxy-2-methyl-2-(4-methylpent-3-en-1-yl)-7-pentyl-*2H*-chromen-6-carboxamide (4k),
[12] (1,1 -dioxidothiomorpholino)(5 -hydroxy-2-methyl-2-(4-methylpent-3 -en-1 -yl)-7-pentyl-2*H*-chromen-6-yl)methanone (41),
[13] 5-hydroxy-2-methyl-2-(4-methylpent-3-en-1-yl)-7-pentyl-*N*-(prop-2-yn-1-yl)-*2H*-chromen-6-carboxamide (4m),
[14] 5-hydroxy-2-methyl-2-(4-methylpent-3-en-1-yl)-7-pentyl-*N*-(3-phenylpropyl)-*2H*-chromen-6-carboxamide (4n),
[15] 5-hydroxy-2-methyl-2-(4-methylpent-3-en-1-yl)-*N*-(1-(methylsulfonyl)piperidin-4-yl)-7-pentyl-2*H*-chromen-6-carboxamide (4o),
[16] *N*-((4-bromothiophen-2-yl)methyl)-5-hydroxy-2-methyl-2-(4-methylpent-3-en-1-yl)-7-pentyl-*2H*-chromen-6-carboxamide (4p),
[17] 5-hydroxy-2-methyl-2-(4-methylpent-3-en-1-yl)-*N*-(1-(methylpiperidin-4-yl)-7-pentyl-*2H*-chromen-6-carboxamide (4q),
[18] 5-hydroxy-2-methyl-2-(4-methylpent-3-en-1-yl)-*N*-(5-methylthiazol-2-yl)-7-pentyl-*2H*-chromen-6-carboxamide (4r),
[19] *tert*-butyl(3S)-3-(5-hydroxy-2-methyl-2-(4-methylpent-3-en-1-yl)-7-pentyl-*2H-*chromen-6-carboxamido)piperidin-1-carboxylate (4s),
[20] 5-hydroxy-2-methyl-2-(4-methylpent-3-en-1-yl)-7-pentyl*-N*-(p-tolyl)-*2H-*chromen-6-carboxamide (4t),
[21] *tert*-butyl 4-(5-hydroxy-2-methyl-2-(4-methylpent-3-en-1-yl)-7-pentyl-*2H-*chromen-6-carboxamido)piperidin-1-carboxylate (4u),
[22] *N*-(*sec*-butyl)-5-hydroxy-2-methyl-2-(4-methylpent-3-en-1-yl)-7-pentyl-*2H-*chromen-6-carboxamide (4v),
[23] *N*-(4'-fluoro-[1,1'-biphenyl]-4-yl)-5-hydroxy-2-methyl-2-(4-methylpent-3-en-1-yl)-7-pentyl-*2H*-chromen-6-carboxamide (4w),
[24] 5-hydroxy-*N*-(4-hydroxyphenethyl)-2-methyl-2-(4-methylpent-3-en-1-yl)-7-pentyl-*2H*-chromen-6-carboxamide (4x),
[25] 5-hydroxy-*N*-(2-mercaptoethyl)-2-methyl-2-(4-methylpent-3-en-1-yl)-7-pentyl-*2H*-chromen-6-carboxamide (4y),
[26] 5-hydroxy-2-methyl-2-(4-methylpent-3-en-1-yl)-7-pentyl-*N*-(piperidin-1-yl)-*2H-*chromen-6-carboxamide (4z),
[27] *N*-(2,3-dihydro-1H-inden-2-yl)-5-hydroxy-2-methyl-2-(4-methylpent-3-en-1-yl)-7-pentyl-*2H*-chromen-6-carboxamide (4aa),
[28] *N*-cycloheptyl-5-hydroxy-2-methyl-2-(4-methylpent-3-en-1-yl)-7-penty1-*2H-*chromen-6-carboxamide (4ab),
[29] (3,4-dihydroisoquinolin-2(1H)-yl)(5-hydroxy-2-methyl-2-(4-methylpent-3-en-1-yl)-7-pentyl-*2H*-chromen-6-yl)methanone (4ac),
[30] *N*-(4-bromophenyl)-5-hydroxy-2-methyl-2-(4-methylpent-3-en-1-yl)-7-pentyl-*2H*-chromen-6-carboxamide (4ad),
[31] (5-hydroxy-2-methyl-2-(4-methylpent-3-en-1-yl)-7-pentyl-*2H*-chromen-6-yl)(4-(2-hydroxyethyl)piperazin-1-yl)methanone (4ae),
[32] 5-hydroxy-2-methyl-2-(4-methylpent-3-en-1-yl)-7-pentyl-*N*-(tetrahydro-2H-pyran-4-yl)-2H-chromen-6-carboxamide (4af),
[33] *N*-cyclopentyl-5-hydroxy-N,2-dimethyl-2-(4-methylpent-3-en-1-yl)-7-pentyl-*2H*-chromen-6-carboxamide (4ag).

This specification uses the following definitions when defining the compound of Formula 1 unless specifically defined.

The term "alkyl" refers to a straight or branched chain hydrocarbonyl group, preferably C₁-C₁₂ alkyl. Examples of alkyl include, but are not limited to, methyl, ethyl, *n*-propyl, *iso*propyl, *n*-butyl, *iso*-butyl, *tert*-butyl, *n*-pentyl, *iso*-pentyl, *n*-hexyl, 3-methylhexyl, 2,2-dimethylpentyl, 2,3-dimethylpentyl, *n*-heptyl, *n*-octyl, *n*-nonyl, *n*-decyl, *n*-undecyl and *n-*dodecyl, etc..

As used herein, the term "alkenyl" refers to a straight-chain or branched-chain unsaturated hydrocarbon having at least one carbon-carbon double bond, preferably containing from 1 to 10 carbon atoms, but not limited thereto.

As used herein, the term "alkynyl" is a straight-chain or branched-chain unsaturated hydrocarbon having at least one triple bond, preferably containing from 1 to 10 carbon atoms, but not limited thereto.

The term "cycloalkyl" refers to a partially or fully saturated single or fused ring hydrocarbon, preferably C₃-C₁₀-cycloalkyl. Examples of cycloalkyl include, but are not limited to, cyclopropyl, cyclobutyl, cyclopentyl, cyclohexyl, and cyclohexenyl.

As used herein, the term "hydroxy" is defined as -OH, the term "oxido" as used herein refers to the =O moiety, and the term "mercapto" or "thiol" refers to -SH group.

As used herein, the term "halogen" or "halo" refers to fluorine (F), chlorine (Cl), bromine (Br) or iodine (I).

The term "aryl" means aromatic hydrocarbon, includes a polycycle aromatic ring system in which a carbocycle aromatic ring or heteroaryl ring is fused with one or more other rings, preferably C₅-C₁₂ aryl, more preferably C₅-C₁₀ aryl. For example, aryl includes, but are not limited to, phenyl, naphthyl, tetrahydronaphthyl, etc..

In addition, there are groups in which the heteroaryl ring is fused to a cycloalkyl or non-aromatic heterocyclic ring, such as indanyl or tetrahydrobenzopyranyl.

The term "heteroaryl" or "aromatic heterocycle" means a 3- to 12-membered, more preferably 5- to 10-membered aromatic hydrocarbon forming a single or fused cyclic ring that contains one or more heteroatoms selected from N, O and S as ring atoms, and that can be fused with a benzo or C₃-C₈ cycloalkyl. For example, heteroaryl includes, but are not limited to, pyrrolyl, imidazolyl, pyrazolyl, triazolyl, pyridyl, pyrazinyl, pyrimidyl, pyridazinyl, triazinyl, oxadiazolyl, isoxadiazolyl, tetrazolyl, indolyl, indazolyl, isoxazolyl, oxazolyl, thiazolyl, isothiazolyl, furanyl, benzofuranyl, thiophenyl, benzothiazolyl, benzooxazolyl, benzimidazolyl, quinolinyl, isoquinolinyl, etc..

A non-aromatic heterocyclic ring is a non-aromatic carbocyclic ring containing one or more heteroatom, such as N, O or S, within the ring. The ring may be 5, 6, 7 or 8-membered and/or fused to another ring such as a cycloalkyl or aromatic ring. Examples of such compounds include 3-1H-benzimidazol-2-one, 3-1-alkyl-benzimidazol-2-one, 2-tetrahydrofuranyl, 3-tetrahydrofuranyl, 2-tetrahydrothiophenyl, 3-tetrahydrothiophenyl, 2-morpholino, 3-morpholino, 4-morpholino, 2-thiomorpholino, 3-thiomorpholino, 4-thiomorpholino, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxic acid, benzotriazol-1-yl, benzopyrrolidine, benzopiperidine, benzoxolane, benzothiolane and benzothiane, etc..

Arylalkyl, alkylaryl and heteroarylalkyl refer to a group formed by combining aryl and alkyl or heteroaryl and alkyl as defined above, and include, for example, benzyl, phenethyl, phenylpropyl etc., but are not limited thereto.

Heterocycloalkyl includes, but is not limited to, tetrahydrofuranyl, thiolenyl, pyrrolidinyl, tetrahydropyranyl, piperidinyl, dioxanyl, morpholino, tetrahydropyranyl or tetrahydropyrimidinyl, etc..

The compound represented by Formula 1 according to the present invention can be prepared and used in the form of prodrugs, hydrates, solvates and pharmaceutically acceptable salts to enhance *in vivo* absorption or increase solubility, so the prodrugs, hydrates, solvates and pharmaceutically acceptable salts are also within the scope of the present invention.

The term "prodrug" refers to a substance that is transformed *in vivo* into a parent drug. Prodrugs are often used because, in some cases, they are easier to administer than the parent drug. For example, they may be bioavailable by oral administration, whereas the parent drug may not be. Prodrugs may also have improved solubility in pharmaceutical compositions than the parent drug. For example, a prodrug may be an *in vivo* hydrolysable ester of the compound according to the present invention and a pharmaceutically acceptable salt thereof. Another example of a prodrug may be a short peptide (poly-amino acid) in which the peptide is coupled to an acid group that is metabolically converted to reveal the active site.

The term "hydrate" refers to a compound of the present invention or a salt thereof containing a stoichiometric or non-stoichiometric amount of water bound by non-covalent intermolecular forces.

The term "solvate" refers to a compound of the present invention or a salt thereof containing a stoichiometric or non-stoichiometric amount of solvent bound by non-covalent intermolecular forces. Preferred solvents therefor include solvents that are volatile, non-toxic, and/or suitable for administration to humans.

The term "isomer" refers to a compound of the present invention or a salt thereof that has the same chemical formula or molecular formula but is structurally or sterically different. Such isomers include both structural isomer such as tautomer, and stereoisomers such as R or S isomers with asymmetric carbon center and geometric isomers (trans, cis). All of these isomers and their mixtures thereof are also included within the scope of the present invention.

The term "pharmaceutically acceptable salt" refers to a salt form of a compound that does not cause serious irritation to the organism to which the compound is administered and does not impair the biological activity and physical properties of the compound. The pharmaceutical salts include an acid addition salt formed by an acid containing a pharmaceutically acceptable anion and forming a non-toxic acid addition salt, for example, inorganic acids such as hydrochloric acid, sulfuric acid, nitric acid, phosphoric acid, hydrobromic acid, hydrogen iodide, etc., organic carbon acids such as tartaric acid, formic acid, citric acid, acetic acid, trichloroacetic acid, trifluoroacetic acid, gluconic acid, benzoic acid, lactic acid, fumaric acid, malic acid, salicylic acid, etc., sulfonic acids such as methanesulfonic acid, ethanesulfonic acid, benzenesulfonic acid, p-toluenesulfonic acid, etc. For example, pharmaceutically acceptable carboxylic acid salts include metal salts or alkaline earth metal salts formed by lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline and triethylamine etc. The compound of Formula 1 according to the present invention can also be converted into its salt by conventional methods.

In addition, the compound represented by the Formula 1 according to the present invention also includes hydrates and solvates that can be prepared therefrom.

The present invention also provides a pharmaceutical composition for cognitive function improvement, comprising the cannabichromenic acid derivative compound represented by the Formula 1, a hydrate thereof, a solvate thereof or a pharmaceutically acceptable salt thereof, as an active ingredient.

The present invention provides a pharmaceutical composition for preventing or treating cognitive function-related disease, comprising the cannabichromenic acid derivative compound represented by the Formula 1, a hydrate thereof, a solvate thereof or a pharmaceutically acceptable salt thereof, as an active ingredient.

The present invention provides a method for preventing or treating cognitive function-related disease, comprising: administering a therapeutically effective amount of the cannabichromenic acid derivative compound represented by the Formula 1, a hydrate thereof, a solvate thereof or a pharmaceutically acceptable salt thereof, to a subject in need thereof.

The present invention also provides a use of the cannabichromenic acid derivative compound represented by the Formula 1, a hydrate thereof, a solvate thereof or a pharmaceutically acceptable salt thereof, in manufacture of a medicament for the purpose of preventing or treating cognitive function-related disease.

The present invention also provides a pharmaceutical composition for cognitive function improvement, characterized in that the cognitive function improvement is preventing or treating degenerative brain disease.

In one embodiment, the degenerative brain disease may be one or more selected from the group consisting of Alzheimer's disease, mild cognitive impairment, stroke and vascular dementia, frontotemporal dementia, Lewy body dementia, Creutzfeldt-Jakob disease, traumatic head injury, syphilis, acquired immunodeficiency syndrome and other viral infections, brain abscess, brain tumor, multiple sclerosis, dementia caused by metabolic diseases, hypoxia, Parkinson's disease, Lou Gehrig's disease, Huntington's disease, Pick's disease, amyotrophic lateral sclerosis, epilepsy, ischemia, stroke, attention deficit hyperactivity disorder, schizophrenia, depression, bipolar disorder, post-traumatic stress disorder, spinal cord injury, and myelitis.

The compounds represented by the Formula 1, and pharmaceutically acceptable salts thereof, show excellent acetylcholinesterase, butyrylcholinesterase and monoamine oxidase inhibitory activity, and thus can be used for cognitive function improvement, and in particular, can be useful in the treatment or prevention of dementia. Accordingly, the compounds of the present invention can simultaneously improve the decrease in acetylcholine levels in brain tissue and the decrease in blood butyrylcholine levels in dementia patients, thereby reducing the dosage of each drug and thus reducing the side effects of dementia treatment.

The pharmaceutical composition of the present invention may comprise a pharmaceutically acceptable diluent or carrier, each of which may be formulated according to conventional methods in the form of powders, granules, tablets, capsules, suspensions, emulsions, syrups, aerosols; topicals; suppositories; and sterile injectable solutions. The pharmaceutically acceptable carriers include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methylhydroxybenzoate, propylhydroxybenzoate, talc, magnesium stearate and mineral oil, and the like. They also include diluents or excipients such as fillers, bulking agents, binders, wetting agents, disintegrating agents, and surfactants. Oral solid dosage forms include tablets, pills, powders, granules, capsules, and the like, which may include at least one excipient, such as starch, calcium carbonate, sucrose, lactose, gelatin, and the like, and may include lubricants such as magnesium stearate and talc. Oral liquid preparations may include suspensions, oral solutions, emulsions, syrups, and the like, and may include diluents such as water and liquid paraffin, wetting agents, sweeteners, flavourings, preservatives, and the like. Parenteral preparations include sterilized aqueous solutions, non-aqueous solvents, suspensions, emulsions, creams, lyophilised preparations, and suppositories; non-aqueous solvents and suspensions include propylene glycol, polyethylene glycol, vegetable oils such as olive oil, and injectable esters such as ethylolate. Substrates for suppositories may be witepsol, macrogol, tween 61, cacao gum, laurin gum, glycerogelatin, etc..

The pharmaceutical compositions of the present invention can be administered to mammals, such as livestock and humans, by various routes, e.g., by oral, dermal, subcutaneous, intramuscular, intravenous, intraperitoneal, rectal, intrauterine, intrathecal or cerebrovascular injection, and topical administration. Accordingly, the compositions of the present invention can be formulated in various forms, such as tablets, capsules, aqueous solutions or suspensions. For oral tablets, a carrier such as lactose, cornstarch, or the like, and a disintegrating agent such as magnesium stearate may be typically added. For oral capsules, lactose and/or dry cornstarch may be used as diluents. If an oral aqueous suspension is desired, the active ingredient may be combined with an emulsifier and/or suspending agent. If necessary, specific sweeteners and/or flavoring agents may be added. For intramuscular, intraperitoneal, subcutaneous and intravenous administration, a sterile solution of the active ingredient is usually prepared, and the pH of the solution should be adjusted and buffered accordingly. For intravenous administration, the total concentration of the solute should be adjusted to impart isotonicity to the preparation.. The composition according to the invention may be in the form of an aqueous solution containing a pharmaceutically acceptable carrier, such as brine with a pH of 7.4. The solution may be introduced into the intramuscular bloodstream of a patient by local injection.

The dosage of the active ingredient in the pharmaceutical composition of the present invention depends on the condition and weight of the patient, the extent of the disease, the formulation of the active ingredient, the route and duration of administration, and may be appropriately adjusted depending on the patient. For example, the active ingredient can be administered at a dose of 0.0001 to 1000 mg/kg per day, preferably 0.01 to 100 mg/kg, and the dose may be administered once or in several divided doses per day. Furthermore, the pharmaceutical composition of the present invention may comprise the active ingredient from 0.001 to 90% by weight, based on the total weight of the composition.

The present invention also provides a composition for inhibiting MAO comprising the cannabichromenic acid derivative compound represented by the above Formula 1, a hydrate thereof, a solvate thereof or a pharmaceutically acceptable salt thereof as an active ingredient.

The present invention also provides a food composition for cognitive function improvement comprising the cannabichromenic acid derivative compound represented by the above Formula 1, a hydrate thereof, a solvate thereof or a pharmaceutically acceptable salt thereof as an active ingredient.

The food composition of the present invention can be used as a health functional food. The term "health functional food" refers to food manufactured and processed using raw materials or ingredients that have functional properties useful to the human body in accordance with the Act on Health Functional Foods, and the term "functional" refers to food consumed for the purpose of obtaining effects useful for health purposes such as nutrient regulation or physiological action on the structure and function of the human body.

The food compositions of the present invention may contain conventional food additives, and their suitability as "food additives" shall be determined according to the standards and criteria for such items in accordance with the general rules and general test methods for Korea Food Additives Code approved by the Ministry of Food and Drug Safety, unless otherwise specified.

The items listed in the "Korea Food Additives Code" above include, for example, chemical compounds such as ketones, glycine, potassium citrate, nicotinic acid, and cinnamic acid; natural additives such as persimmon coloring, licorice extract, crystalline cellulose, macrocycles, and guar gum; monosodium L-glutamate preparations; and mixed preparations such as noodle alkalis, preservatives, and tar colorants.

The food compositions of the present invention may comprise from 0.01 to 95%, preferably from 1 to 80% by weight, of the compound represented by Formula 1, relative to the total weight of the composition, for the purpose of improving cognitive function, in particular for the prevention and/or improvement of dementia, depression, Parkinson's disease. It can also be prepared and processed in the form of tablets, capsules, powders, granules, liquids, pills, etc. for the purpose of improving cognitive function, in particular for the prevention and/or improvement of dementia, depression, Parkinson's disease.

The present invention also provides a method for preparing a cannabichromenic acid derivative compound represented by the following Formula 1, comprising: reacting (E)-non-3-en-2-one represented by Formula 1-1 below with dimethyl malonate represented by Formula 1-2 below in an organic solvent to obtain a first compound represented by Formula 1a below; mixing the first compound obtained above with citral and calcium hydroxide, reacting in an organic solvent to obtain a second compound represented by Formula 2a below; reacting the second compound obtained above with thiophenol and cesium carbonate to obtain a third compound represented by Formula 3a; and reacting the third compound obtained above with 1-hydroxybenzotriazole, 1-(3-dimethylaminopropy)-3-ethylcarbodiimide hydrochloride and an amine compound, followed by purification to obtain a cannabichromenic acid derivative compound represented by Formula 1 below. 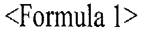 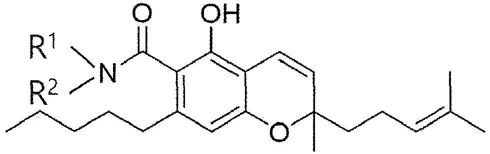 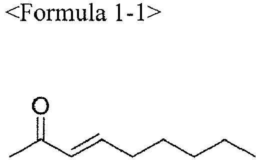 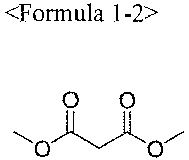 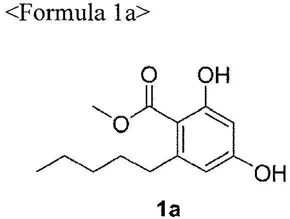 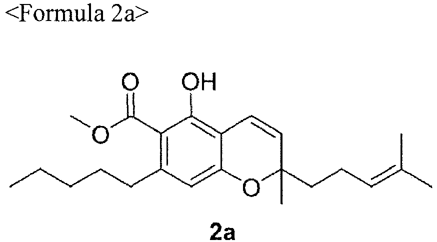 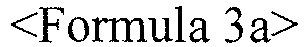 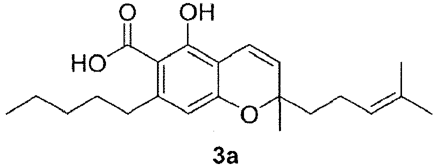 wherein in Formula 1, R¹ and R² are the same as defined in Claim 1.

Scheme 1 to 4 of the Synthesis Examples are exemplified as processes for synthesizing the compounds of Formula 1 of the present invention, and the methods of preparation of Scheme 1 to 4 are not intended to be limiting to methods for preparing the compounds of Formula 1 according to the present invention. It will be appreciated that the method of preparation of Formula 1 is exemplary only and can be readily modified by one of ordinary skill in the art depending on specific substituents.

Hereinafter, the present invention will be described in more detail with Synthesis Examples, Examples and Experimental examples. However, the following Synthesis Examples, Examples and Experimental examples are intended to illustrate the present invention and are not intended to limit the scope of the present invention.

### Synthesis Example 1. General procedure for the synthesis of methyl 2,4-dihydroxy-6-pentylbenzoate (1a)

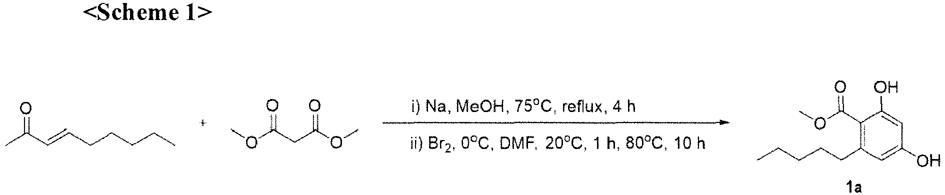

In a 100 mL round bottom flask, sodium piece (1.03 g, 44.6 mmol) was completely dissolved in methanol (11.9 mL) maintained at 0 °C, to which was added dimethyl malonate (5.76 mL, 50.4 mmol) and (E)-non-3-en-2-one (5.95 mL, 36.0 mmol), reflux cooled and stirred at 75 °C for 4 hours. When the reaction was complete, as confirmed by thin-layer chromatography (TLC), methanol was removed by evaporation under reduced pressure. The remaining reaction mixture was diluted with water and chloroform (CHCl₃), acidified to pH 3.0 by adding 1 N aqueous hydrogen chloride solution, and extracted three times with chloroform. The extracted organic layer was dried over magnesium sulfate and re-dissolved in N,N-dimethylformamide (DMF, 21.3 ml) at 0 °C. To this was slowly added 9.2 mL of a 1.84 mL solution of bromine (Br₂) diluted 1:4 in DMF and stirred at 20 °C for 1 hour. The reaction mixture was stirred at 80 °C for 10 h. When the reaction was complete, as confirmed by TLC, the solvent was evaporated under reduced pressure. The organic layer obtained by extraction with 5% aqueous sodium thiosulfate and ethyl acetate was dried over magnesium sulfate and purified by column chromatography to give a yellow solid compound, (silica gel, ethyl acetate/hexane = 1:20, v/v) (0.51 g, 61% yield)

### Synthesis Example 2. General procedure for the synthesis of methyl 5-hydroxy-2-methyl-2-(4-methylpent-3-en-1-yl)-7-pentyl-2H-chromene-6-carboxylate (2a)

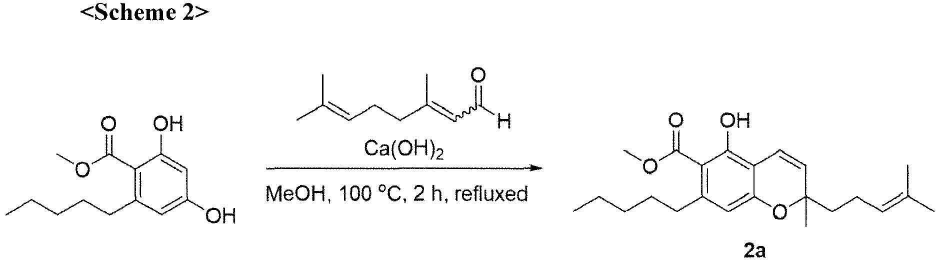

In a 100 mL round bottom flask, citral (14.26 mL, 66.0 mmol), 2,4-dihydroxy-6-pentylbenzoate (5.22 g, 22.0 mmol) and calcium hydroxide (1.63 g, 22.0 mmol) were dissolved in 44 mL of methanol, then the flask was sealed, refluxed and stirred at 100 °C for 2 h. When the reaction was complete, as confirmed by TLC, the methanol was evaporated under reduced pressure. The mixture was acidified to pH 3.0 with 1 N aqueous hydrogen chloride solution, and the organic layer was extracted using ethyl acetate. The extracted organic layer was dried over magnesium sulfate and purified by column chromatography to give a colorless oil-like compound. (silica gel, ethyl acetate/hexane = 1 : 50, v/v) (0.52 g, 61% yield)

### Synthesis Example 3. General procedure for the synthesis of 5-hydroxy-2-methyl-2-(4-methylpent-3-en-1-yl)-7-pentyl-2H-chromene-6-carboxylic acid (CBCA) (3a)

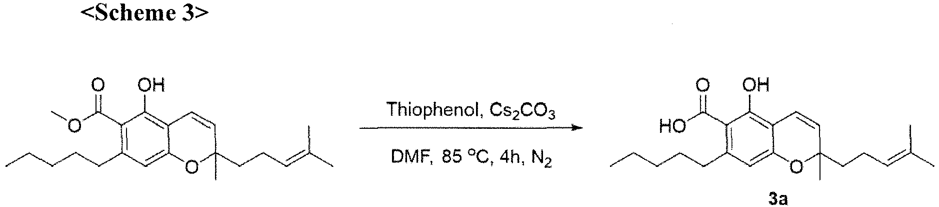

In a 50 mL round bottom flask, the synthesized methyl ester compound (1.12 g, 3.0 mmol) and cesium carbonate (0.49 g, 1.5 mmol) were dissolved in 12 mL of DMF, then thiophenol (462.1 µl, 4.5 mmol) was slowly added, sealed and stirred at 85 °C under nitrogen for 4 hours. When the reaction was complete, as confirmed by TLC, DMF was evaporated under reduced pressure. The reaction product was acidified to pH 3.0 with 1 N aqueous hydrogen chloride solution, and the organic layer was extracted with ethyl acetate. The extracted organic layer was dried over magnesium sulfate and purified by column chromatography to give a yellow solid compound, (silica gel, ethyl acetate/hexane = 1 : 7 v/v) (0.97 g, 90% yield)

### Synthesis Example 4. General procedure for the synthesis of N-cyclopropyl-5-hydroxy-2-methyl-2-(4-methylpent-3-en-1-yl)-7-pentyl-2H-chromene-6-carboxamide (Formula 1)

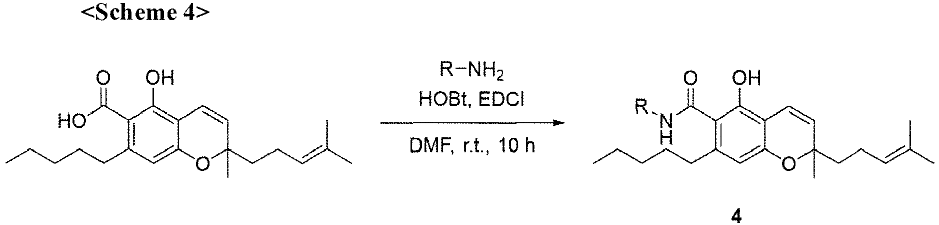

In a 10 mL round bottom flask, cannabichromenic acid (CBCA, 107.5 mg, 0.3 mmol), 1-hydroxybenzotriazole (HOBt, 135.1 mg, 0.9 mmol), 1-(3-dimethylaminopropy)-3-ethylcarbodiimide hydrochloride (EDCI, 172. 5 mg, 0.9 mmol) were dissolved in 2.1 mL of DMF, and then stirred at room temperature for 1 hour. 0.36 mmol of 19 different amine compounds were added to the formed intermediate, followed by stirring at room temperature for 10 hours. Upon completion of the reaction, water was added to terminate the reaction, and the organic layer was extracted with dichloromethane (CH₂Cl₂). The residual DMF was evaporated and then the extracted organic layer was dried over magnesium sulfate and purified by column chromatography to give an oil compound (silica gel, ethyl acetate/hexane = 1:30, v/v).

### Example 1. 5-hydroxy-N-isopropyl-2-methyl-2-(4-methylpent-3-en-1-yl)-7-pentyl-2H-chromene-6-carboxamide (4a)

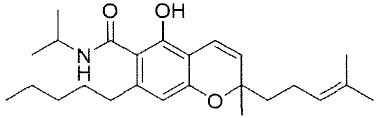

The compound was prepared according to Scheme 4 using cannabichromenic acid (CBCA) (3a) as starting material. It was purified by column chromatography on silica gel, with ethyl acetate/hexane = 1 : 50, v/v to give a brown oil (37.0 mg, 21% yield).

¹H NMR (400 MHz, CDCl3) δ 11.28 (s, 1H), 6.72 (d, J = 10.10 Hz, 1H), 6.18 (s, 1H), 5.74 (d, J = 6.76 Hz, 1H), 5.47 (d, J = 10.06 Hz, 1H), 5.10 - 5.06 (m, 1H), 4.31 - 4.26 (m, 1H), 2.67 (t, J = 8.10 Hz, 2H), 2.11 - 2.07 (m, 2H), 1.74 - 1.58 (m, 8H), 1.57 (s, 3H), 1.37 (s, 3H), 1.36 - 1.32 (m, 4H), 1.26 (s, 3H), 1.24 (s, 3H), 0.92 - 0.89 (m, 3H).

### Example 2. 5-hydroxy-2-methyl-2-(4-methylpent-3-en-1-yl)-7-pentyl-N-phenyl-2H-chromene-6-carboxamide (4b)

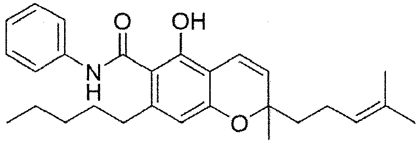

The compound was prepared according to Scheme 4 using cannabichromenic acid (CBCA) (3a) as starting material. It was purified by column chromatography on silica gel, with ethyl acetate/hexane = 1:30, v/v to give a yellow oil (22.2 mg, 13% yield).

¹H NMR (400 MHz, CDCl₃) δ 10.73 (s, 1H), 7.53 (d, J = 7.52 Hz, 2H), 7.41 - 7.37 (m, 2H), 7.19 (t, J = 7.42 Hz, 1H) 6.74 (d, J = 10.06 Hz, 1H), 6.27 (s, 1H), 5.51 (d, J = 10.08 Hz, 1H), 5.11 - 5.08 (m, 1H), 2.82 - 2.78 (m, 2H), 2.13 - 2.07 (m, 2H), 1.79 - 1.61 (m, 7H), 1.57 (s, 3H), 1.40 (s, 3H), 1.39 - 1.33 (m, 4H), 0.88 (t, J = 7.08 Hz, 3H).

### Example 3. N-cyclopropyl-5-hydroxy-2-methyl-2-(4-methylpent-3-en-1-yl)-7-pentyl-2H-chromene-6-carboxamide (4c)

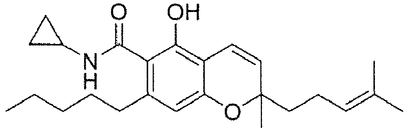

The compound was prepared according to Scheme 4 using cannabichromenic acid (CBCA) (3a) as starting material. It was purified by column chromatography on silica gel, with ethyl acetate/hexane = 1:15, v/v to give a colorless transparent oil (24.3 mg, 20% yield).

¹H NMR (400 MHz, CDCl₃) δ 11.37 (s, 1H), 6.72 (d, J = 10.08 Hz, 1H), 6.17 (s, 1H), 6.02 (s, 1H), 5.48 (d, J = 10.08 Hz, 1H), 5.08 (t, J = 7.16 Hz, 1H), 2.93 - 2.87 (m, 1H), 2.64 - 2.60 (m, 2H), 2.08 (q, J = 7.90 Hz, 2H), 1.76 - 1.58 (m, 2H), 1.65 (s, 3H), 1.57 (s, 3H), 1.38 (s, 3H), 1.35 - 1.31 (m, 4H), 0.93 - 0.87 (m, 5H), 0.61 - 0.57 (m, 2H).

### Example 4. N-butyl-5-hydroxy-2-methyl-2-(4-methylpent-3-en-1-yl)-7-pentyl-2H-chromene-6-carboxamide (4d)

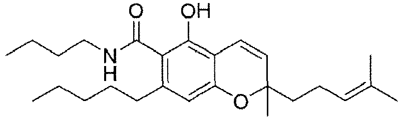

The compound was prepared according to Scheme 4 using cannabichromenic acid (CBCA) (3a) as starting material. It was purified by column chromatography on silica gel, with ethyl acetate/hexane = 1:20, v/v to give a colorless, transparent oil (30.5 mg, 24% yield).

¹H NMR (400 MHz, CDCl₃) δ 11.35 (s, 1H), 6.72 (d, J = 10.08 Hz, 1H), 6.19 (s, 1H), 5.87 (s, 1H), 5.48 (d, J = 10.08 Hz, 1H), 5.09 (t, J = 7.16 Hz, 1H), 4.05 - 3.96 (m, 1H), 2.70 - 2.66 (m, 2H), 2.11 - 2.00 (m, 4H), 1.77 - 1.58 (m, 2H), 1.66 (s, 3H), 1.57 (s, 3H), 1.46 - 1.40 (m, 2H), 1.38 (s, 3H), 1.37 - 1.32 (m, 2H), 0.97 (t, J = 7.34 Hz, 3H), 0.91 (t, J = 7.09 Hz, 3H)

### Example 5. N-cyclopentyl-5-hydroxy-2-methyl-2-(4-methylpent-3-en-1-yl)-7-pentyl-2H-chromene-6-carboxamide (4e)

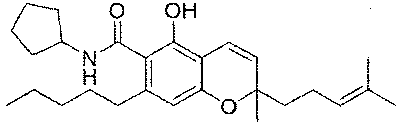

The compound was prepared according to Scheme 4 using cannabichromenic acid (CBCA) (3a) as starting material. It was purified by column chromatography on silica gel, with ethyl acetate/hexane = 1:20, v/v to give a colorless, transparent oil (31.1 mg, 23% yield).

¹H NMR (400 MHz, CDCl₃) δ 11.38 (s, 1H), 6.72 (d, J = 10.12 Hz, 1H), 6.18 (s, 1H), 5.87 (d, J = 6.38 Hz, 1H), 5.48 (d, J = 10.08 Hz, 1H), 5.08 (t, J = 7.16 Hz, 1H), 4.45 - 4.37 (m, 1H), 2.69 - 2.65 (m, 2H), 2.11 - 2.03 (m, 4H), 1.77 - 1.68 (m, 5H), 1.66 (s, 3H), 1.64 - 1.59 (m, 3H), 1.57 (s, 3H), 1.51 - 1.47 (m, 2H), 1.38 (s, 3H), 1.36 - 1.33 (m, 4H), 0.91 (t, J = 7.05 Hz, 3H).

### Example 6. N-cyclohexyl-5-hydroxy-2-methyl-2-(4-methylpent-3-en-1-yl)-7-pentyl-2H-chromene-6-carboxamide (4f)

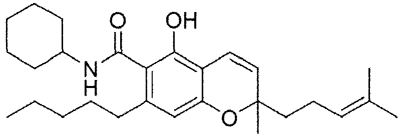

The compound was prepared according to Scheme 4 using cannabichromenic acid (CBCA) (3a) as starting material. It was purified by column chromatography on silica gel, with ethyl acetate/hexane = 1:20, v/v to give a colorless transparent oil (13.8 mg, 9% yield).

¹H NMR (400 MHz, CDCl₃) δ 11.30 (s, 1H), 6.72 (d, J = 10.00 Hz, 1H), 6.18 (s, 1H), 5.80 (d, J = 7.72 Hz, 1H), 5.48 (d, J = 10.08 Hz, 1H), 5.08 (t, J = 7.12 Hz, 1H), 4.05 - 3.96 (m, 1H), 2.70 - 2.66 (m, 2H), 2.11 -2.00 (m, 4H), 1.77 - 1.69 (m, 4H), 1.66 (s, 3H), 1.64 - 1.59 (m, 4H), 1.57 (s, 3H), 1.46 - 1.42 (m, 2H), 1.38 (s, 3H), 1.36 - 1.34 (m, 4H), 1.24 - 1.21 (m, 2H), 0.91 (t, J = 7.07 Hz, 3H).

### Example 7. 5-hydroxy-2-methyl-2-(4-methylpent-3-en-1-yl)-N-nonyl-7-pentyl-2H-chromene-6-carboxamide (4g)

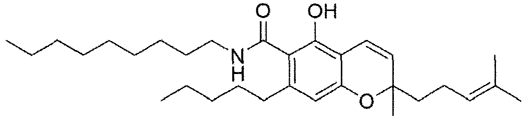

The compound was prepared according to Scheme 4 using cannabichromenic acid (CBCA) (3a) as starting material. It was purified by column chromatography on silica gel, with ethyl acetate/hexane = 1 : 30, v/v to give a colorless transparent oil (37.7 mg, 25% yield).

¹H NMR (400 MHz, CDCl₃) δ 11.36 (s, 1H), 6.72 (d, J = 10.06 Hz, 1H), 6.19 (s, 1H), 5.88 (s, 1H), 5.48 (d, J = 10.08 Hz, 1H), 5.09 (t, J = 7.08 Hz, 1H), 3.44 (q, J = 6.60 Hz, 2H), 2.70 - 2.66 (m, 2H), 2.09 (q, J = 7.76 Hz, 2H), 1.75 - 1.69 (m, 1H), 1.67 (s, 3H), 1.64 - 1.60 (m, 5H), 1.57 (s, 3H), 1.38 (s, 3H), 1.37 - 1.27 (m, 16H), 0.92 - 0.84 (m, 6H).

### Example 8. N-dodecyl-5-hydroxy-2-methyl-2-(4-methylpent-3-en-1-yl)-7-pentyl-2H-chromene-6-carboxamide (4h)

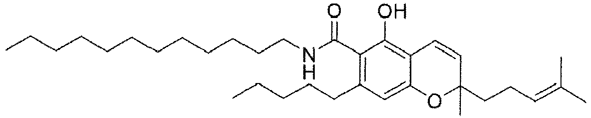

The compound was prepared according to Scheme 4 using cannabichromenic acid (CBCA) (3a) as starting material. It was purified by column chromatography on silica gel, with ethyl acetate/hexane = 1 : 30, v/v to give a colorless transparent oil (19.0 mg, 10% yield).

¹H NMR (400 MHz, CDCl₃) δ 11.36 (s, 1H), 6.72 (d, J = 10.11 Hz, 1H), 6.19 (s, 1H), 5.88 (s, 1H), 5.48 (d, J = 10.08 Hz, 1H), 5.09 (t, J = 7.21 Hz, 1H), 3.44 (q, J = 6.58 Hz, 2H), 2.71 - 2.66 (m, 2H), 2.12 - 2.06 (q, J = 7.95 Hz, 2H), 1.77 - 1.69 (m, 1H), 1.66 (s, 3H), 1.64 - 1.60 (m, 5H), 1.57 (s, 3H), 1.38 (s, 3H), 1.37 - 1.30 (m, 6H), 1.26 (s, 16H), 0.92 - 0.86 (m, 6H).

### Example 9. 5-hydroxy-2-methyl-2-(4-methylpent-3-en-1-yl)-7-pentyl-N-(undec-10-en-1-yl)-2H-chromene-6-carboxamide (4i)

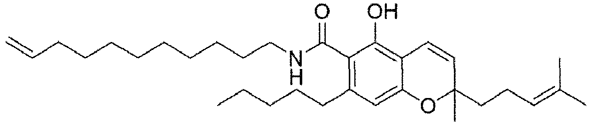

The compound was prepared according to Scheme 4 using cannabichromenic acid (CBCA) (3a) as starting material. It was purified by column chromatography on silica gel, with ethyl acetate/hexane = 1 : 50, v/v to give a colorless transparent oil (50.9 mg, 28% yield).

¹H NMR (400 MHz, CDCl₃) δ 11.36 (s, 1H), 6.72 (d, J = 10.06 Hz, 1H), 6.19 (s, 1H), 5.88 (s, 1H), 5.86 - 5.76 (m, 1H), 5.48 (d, J = 10.06 Hz, 1H), 5.09 (t, J = 7.24 Hz, 1H). 5.02 - 4.96 (m, 1H), 4.94 - 4.92 (m, 1H), 3.44 (q, J = 6.58 Hz, 2H), 2.70 - 2.66 (m, 2H), 2.11 - 2.01 (m, 4H), 1.77 - 1.57 (m, 13H), 1.38 - 1.25 (m, 22H), 0.91 (t, J = 7.05 Hz, 3H).

### Example 10. N-(2-ethylhexyl)-5-hydroxy-2-methyl-2-(4-methylpent-3-en-1-yl)-7-pentyl-2H-chromene-6-carboxamide (4j)

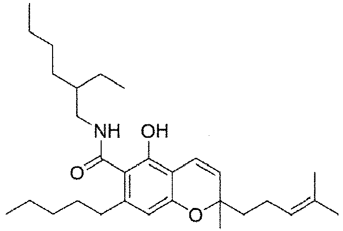

The compound was prepared according to Scheme 4 using cannabichromenic acid (CBCA) (3a) as starting material. It was purified by column chromatography on silica gel, with ethyl acetate/hexane = 1:30, v/v to give a colorless transparent oil (19.9 mg, 12% yield).

¹H NMR (400 MHz, CDCl₃) δ 11.43 (s, 1H), 6.73 (d, J = 10.06 Hz, 1H), 6.20 (s, 1H), 5.84 (s, 1H), 5.48 (d, J = 10.07 Hz, 1H), 5.09 (t, J = 7.24 Hz, 1H). 3.46 - 3.36 (m, 2H), 2.71 - 2.67 (m, 2H), 2.09 (q, J = 7.71 Hz, 2H), 1.77 - 1.57 (m, 13H), 1.38 - 1.29 (m, 15H), 0.97 - 0.89 (m, 9H).

### Example 11. (E)-N-(3,7-dimethylocta-2,6-dien-1-yl)-5-hydroxy-2-methyl-2-(4-methylpent-3-en-1-yl)-7-pentyl-2H-chromene-6-carboxamide (4k)

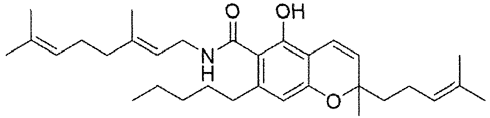

The compound was prepared according to Scheme 4 using cannabichromenic acid (CBCA) (3a) as starting material. It was purified by column chromatography on silica gel, with ethyl acetate/hexane = 1 : 30, v/v to give a colorless transparent oil (11.0 mg, 6% yield).

¹H NMR (400 MHz, CDCl₃) δ 11.41 (s, 1H), 6.73 (d, J = 10.18 Hz, 1H), 6.18 (s, 1H), 5.77 (s, 1H), 5.48 (d, J = 10.09 Hz, 1H), 5.31 - 5.27 (m, 1H), 5.09 (t, J = 6.92 Hz, 2H), 4.05 - 4.02 (m, 2H), 2.69 - 2.65 (m, 2H), 2.11 - 2.04 (m, 6H), 1.74 - 1.57 (m, 19H), 1.38 (s, 3H), 1.35 - 1.29 (m, 15H), 0.90 (t, J = 7.01 Hz, 3H).

### Example 12. (1,1-dioxidothiomorpholino)(5-hydroxy-2-methyl-2-(4-methylpent-3-en-1-yl)-7-pentyl-2H-chromen-6-yl)methanone (4l)

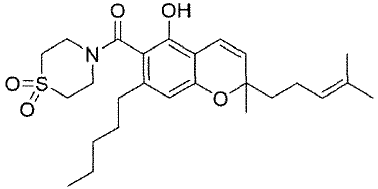

The compound was prepared according to Scheme 4 using cannabichromenic acid (CBCA) (3a) as starting material. It was purified by column chromatography on silica gel, with ethyl acetate/hexane = 1:3, v/v to give a colorless transparent oil (80.3 mg, 38% yield).

¹H NMR (400 MHz, CDCl₃) δ 6.57 (d, J = 10.08 Hz, 1H), 6.27 (s, 1H), 5.52 (d, J = 10.09 Hz, 1H), 5.09 (m, 1H), 5.09 (t, J = 7.09 Hz, 1H), 4.39 (br s, 1H), 3.69 (t, J = 12.64 Hz, 2H), 3.26 - 3.17 (m, 2H), 2.99 - 2.96 (m, 2H), 2.40 (t, J = 7.88 Hz, 2H), 2.14 - 2.04 (m, 2H), 1.76 - 1.58 (m, 10H), 1.54 - 1.47 (m, 2H), 1.36 (s, 3H), 1.31 - 1.27 (m, 4H), 0.88 (t, J = 6.93 Hz, 3H).

### Example 13. 5-hydroxy-2-methyl-2-(4-methylpent-3-en-1-yl)-7-pentyl-N-(prop-2-yn-1-yl)-2H-chromene-6-carboxamide (4m)

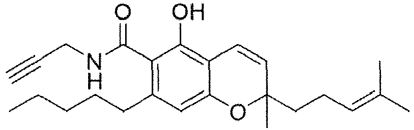

The compound was prepared according to Scheme 4 using cannabichromenic acid (CBCA) (3a) as starting material. It was purified by column chromatography on silica gel, with ethyl acetate/hexane = 1:30, v/v to give a colorless transparent oil (34.0 mg, 21% yield).

¹H NMR (400 MHz, CDCl₃) δ 11.22 (s, 1H), 6.72 (d, J = 10.12 Hz, 1H), 6.20 (s, 1H), 6.05 (s, 1H), 5.49 (d, J = 10.08 Hz, 1H), 5.09 (t, J = 7.12 Hz, 1H), 4.25 - 4.23 (m, 2H), 2.73 - 2.69 (m, 2H), 2.30 (t, J = 2.56 Hz, 1H), 2.08 (q, J = 7.92 Hz, 2H), 1.77 - 1.59 (m, 10H), 1.57 (s, 3H), 1.39 (s, 3H), 1.37 - 1.35 (m, 4H), 0.91 (t, J = 7.03 Hz, 3H).

### Example 14. 5-hydroxy-2-methyl-2-(4-methylpent-3-en-1-yl)-7-pentyl-N-(3-phenylpropyl)-2H-chromene-6-carboxamide (4n)

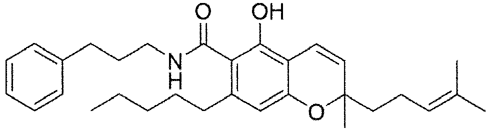

The compound was prepared according to Scheme 4 using cannabichromenic acid (CBCA) (3a) as starting material. It was purified by column chromatography on silica gel, with ethyl acetate/hexane = 1 : 30, v/v to give a yellow oil (17.5 mg, 4% yield).

¹H NMR (400 MHz, CDCl₃) δ 11.32 (s, 1H), 7.31 - 7.27 (m, 2H), 7.22 - 7.18 (m, 3H), 6.72 (d, J = 10.08 Hz, 1H), 6.19 (s, 1H), 5.89 - 5.87 (m, 1H), 5.48 (d, J = 10.08 Hz, 1H), 5.09 (t, J = 7.16 Hz, 1H), 3.48 (q, J = 6.67Hz, 2H), 2.72 (t, J = 7.66 Hz, 2H), 2.68 - 2.64 (m, 2H) 2.14 - 2.06 (m, 2H), 1.98 - 1.91 (m, 2H), 1.77 - 1.59 (m, 10H), 1.57 (s, 3H), 1.38 (s, 3H), 1.37 - 1.32 (m, 4H), 0.88 (t, J = 7.05 Hz, 3H).

### Example 15. 5-hydroxy-2-methyl-2-(4-methylpent-3-en-1-yl)-N-(1-(methylsulfonyl)piperidin-4-yl)-7-pentyl-2H-chromene-6-carboxamide (4o)

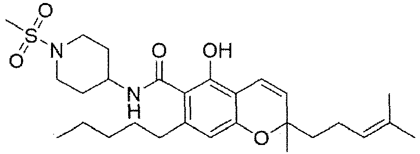

The compound was prepared according to Scheme 4 using cannabichromenic acid (CBCA) (3a) as starting material. It was purified by column chromatography on silica gel, with ethyl acetate/hexane = 1 : 20, v/v to give a white solid (24.3 mg, 20% yield).

¹H NMR (400 MHz, CDCl₃) δ 11.16 (s, 1H), 6.71 (d, J = 10.08 Hz, 1H), 6.20 (s, 1H), 5.23 (d, J = 7.88 Hz, 1H), 5.49 (d, J = 10.08 Hz, 1H), 5.08 (t, J = 7.16 Hz, 1H), 4.16 - 4.09 (m, 1H), 3.84 (d, J = 12.33 Hz, 2H), 2.89 - 2.82 (m, 2H), 2.81 (s, 1H), 2.69 - 2.65 (m, 2H), 2.17 (d, J = 11.75 Hz, 2H), 2.08 (q, J = 7.93 Hz, 2H), 1.77 - 1.57 (m, 16H), 1.38 (s, 3H), 1.36 - 1.34 (m, 4H), 0.93 -0.89 (m, 3H).

### Example 16. N-((4-bromothiophen-2-yl)methyl)-5-hydroxy-2-methyl-2-(4-methylpent-3-en-1-yl)-7-pentyl-2H-chromene-6-carboxamide (4p)

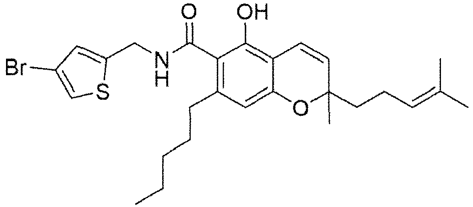

The compound was prepared according to Scheme 4 using cannabichromenic acid (CBCA) (3a) as starting material. It was purified by column chromatography on silica gel, with ethyl acetate/hexane = 1 : 50, v/v to give a pale yellow solid (46.0 mg, 31% yield).

¹H NMR (400 MHz, CDCl₃) δ 11.22 (s, 1H), 7.16 (d, J = 1.44 Hz, 1H), 6.96 - 6.95 (m, 1H), 6.72 (d, J = 10.08 Hz, 1H), 6.21 - 6.19 (m, 2H), 5.49 (d, J = 10.12 Hz, 1H), 5.08 (t, J = 7.15 Hz, 1H), 4.75 (d, J = 5.60 Hz, 2H), 2.67 - 2.63 (m, 2H), 2.11 - 2.05 (m, 2H), 1.77 - 1.53 (m, 10H), 1.38 (s, 3H), 1.27 - 1.22 (m, 4H), 0.87 (t, J = 6.88 Hz, 3H).

### Example 17. 5-hydroxy-2-methyl-2-(4-methylpent-3-en-1-yl)-N-(1-methylpiperidin-4-yl)-7-pentyl-2H-chromene-6-carboxamide (4q)

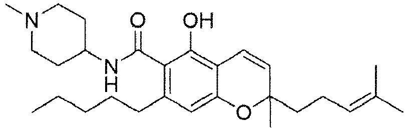

The compound was prepared according to Scheme 4 using cannabichromenic acid (CBCA) (3a) as starting material. It was purified by column chromatography on silica gel, with ethyl acetate/hexane = 1 : 60, v/v to give a yellow oil (30.5 mg, 24% yield).

¹H NMR (400 MHz, CDCl₃) δ 6.71 (d, J = 10.08 Hz, 1H), 6.19 (s, 1H), 5.84 (d, J = 7.33 Hz, 1H), 5.48 (d, J = 10.08 Hz, 1H), 5.10 - 5.06 (m, 1H), 4.04 - 4.02 (m, 1H), 2.90 - 2.87 (m, 2H), 2.67 (t, J = 8.00 Hz, 2H), 2.35 (s, 3H), 2.27 - 2.22 (m, 2H), 2.11 - 2.05 (m, 4H), 1.76 - 1.67 (m, 2H), 1.65 (s, 3H), 1.64 - 1.58 (m, 4H), 1.57 (s, 3H), 1.38 (s, 3H), 1.36 - 1.32 (m, 4H), 0.91 - 0.88 (m, 3H).

### Example 18. 5-hydroxy-2-methyl-2-(4-methylpent-3-en-1-yl)-N-(5-methylthiazol-2-yl)-7-pentyl-2H-chromene-6-carboxamide (4r)

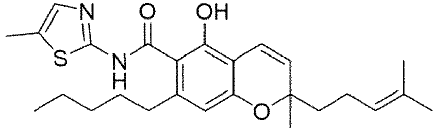

The compound was prepared according to Scheme 4 using cannabichromenic acid (CBCA) (3a) as starting material. It was purified by column chromatography on silica gel, with ethyl acetate/hexane = 1 : 50, v/v to give a yellow oil (16.9 mg, 9% yield).

¹H NMR (400 MHz, CDCl₃) δ 6.74 (d, J = 9.85 Hz, 2H), 6.30 (s, 1H), 5.53 (d, J = 10.09 Hz, 1H), 5.12 - 5.08 (m, 1H), 4.25 - 4.23 (m, 2H), 2.82 (m, J = 7.80 Hz, 2H), 2.36 (s, 1H), 2.14 - 2.08 (m, 2H) 1.78 - 1.56 (m, 10H), 1.42 (s, 3H), 1.31 - 1.27 (m, 4H), 0.84 (t, J = 6.96 Hz, 3H).

### Example 19. tert-butyl(3S)-3-(5-hydroxy-2-methyl-2-(4-methylpent-3-en-1-yl)-7-pentyl-2H-chromene-6-carboxamido)piperidine-1-carboxylate (4s)

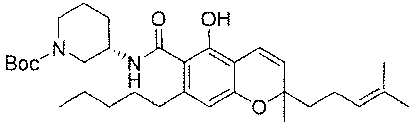

The compound was prepared according to Scheme 4 using cannabichromenic acid (CBCA) (3a) as starting material. It was purified by column chromatography on silica gel, with ethyl acetate/hexane = 1:10, v/v to give a yellow oil (16.0 mg, 7% yield).

¹H NMR (400 MHz, CDCl₃) δ 11.35 (s, 1H), 6.72 (d, J = 10.21 Hz, 1H), 6.19 (s, 1H), 6.03 (brs, 1H), 5.48 (d, J = 10.09 Hz, 1H), 5.10 - 5.06 (m, 1H), 4.18 (brs, 1H), 3.58 - 3.54 (m, 1H), 3.52 - 3.44 (m, 2H), 3.34 - 3.27 (m, 1H), 2.70 - 2.66 (m, 2H), 2.09 - 2.05 (m, 2H), 1.76 - 1.70 (m, 3H), 1.65 (s, 3H), 1.64 - 1.61 (m, 4H), 1.57 (s, 3H), 1.42 (s, 9H), 1.38 (s, 3H), 1.35 - 1.32 (m, 4H), 0.91 - 0.88 (m, 3H).

### Example 20. 5-hydroxy-2-methyl-2-(4-methylpent-3-en-1-yl)-7-pentyl-N-(p-tolyl)-2H-chromene-6-carboxamide (4t)

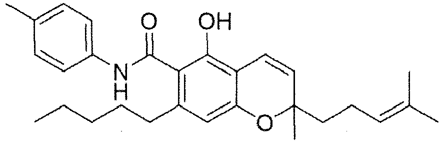

The compound was prepared according to Scheme 4 using cannabichromenic acid (CBCA) (3a) as starting material. It was purified by column chromatography on silica gel, with ethyl acetate/hexane = 1:20, v/v to give a yellow oil (11.0 mg, 7% yield).

¹H NMR (400 MHz, CDCl₃) δ 7.46 (d, J = 8.40 Hz, 2H), 7.13 (d, J = 8.22 Hz, 2H), 6.59 (s, 1H), 6.28 (d, J = 10.02 Hz, 1H), 5.88 (d, J = 10.03 Hz, 1H), 5.10 - 5.07 (m, 1H), 2.63 (t, J = 7.90 Hz, 2H), 2.32 (s, 3H), 2.13 - 2.06 (m, 2H), 1.77 - 1.68 (m, 1H), 1.66 (s, 3H), 1.64 - 1.60 (m, 3H), 1.58 (s, 3H), 1.39 (s, 3H), 1.32 - 1.27 (m, 4H), 0.86 - 0.83 (m, 3H).

### Example 21. tert-butyl 4-(5-hydroxy-2-methyl-2-(4-methylpent-3-en-1-yl)-7-pentyl-2H-chromene-6-carboxamido)piperidine-1-carboxylate (4u)

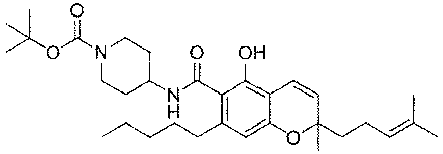

The compound was prepared according to Scheme 4 using cannabichromenic acid (CBCA) (3a) as starting material. It was purified by column chromatography on silica gel, with methanol/dichloromethane = 1 : 80, v/v to give a clear oil (56.0 mg, 26% yield).

¹H NMR (400 MHz, CDCl₃) δ 6.72 (d, J = 10.1 Hz, 1H), 6.19 (s, 1H), 5.80 (d, J = 7.7 Hz, 1H), 5.49 (d, J = 10.1 Hz, 1H), 5.10 - 5.06 (m, 1H), 4.17 - 4.07 (m, 3H), 2.94 - 2.91 (m, 2H), 2.67 (t, J = 8.0 Hz, 2H), 2.10 - 2.01 (m, 4H), 1.74 - 1.57(m, 12H), 1.47 (s, 9H), 1.38 (s, 3H), 1.36 - 1.29 (m, 4H), 0.93 - 0.88 (m, 3H).

### Example 22. N-(sec-butyl)-5-hydroxy-2-methyl-2-(4-methylpent-3-en-1-yl)-7-pentyl-2H-chromene-6-carboxamide (4v)

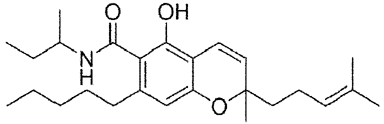

The compound was prepared according to Scheme 4 using cannabichromenic acid (CBCA) (3a) as starting material. It was purified by column chromatography on silica gel, with ethyl acetate/hexane = 1:20, v/v to give a yellow oil (42.0 mg, 25% yield).

¹H NMR (400 MHz, CDCl₃) δ 11.31(s, 1H), 6.72 (d, J = 10.0 Hz, 1H), 6.19 (s, 1H), 5.70 (d, J = 8.0 Hz, 1H), 5.48 (d, J = 10.1 Hz, 1H), 5.08 (t, J = 7.2 Hz, 1H), 4.16 - 4.11 (m, 1H), 2.68 (t, J = 8.1 Hz, 2H), 2.12 - 2.04 (m, 2H), 1.76 - 1.56 (m, 15H), 1.38 (s, 3H), 1.37 - 1.31 (m, 4H), 1.24 (t, J = 6.9 Hz, 3H), 1.00 - 0.94 (m, 3H), 0.93 - 0.87 (m, 3H).

### Example 23. N-(4'-fluoro-[1,1'-biphenyl]-4-yl)-5-hydroxy-2-methyl-2-(4-methylpent-3-en-1-yl)-7-pentyl-2H-chromene-6-carboxamide (4w)

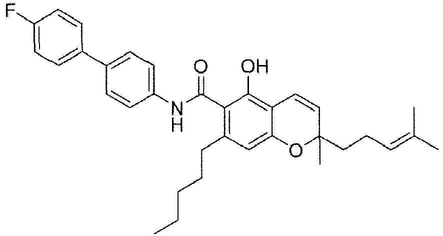

The compound was prepared according to Scheme 4 using cannabichromenic acid (CBCA) (3a) as starting material. It was purified by column chromatography on silica gel, with ethyl acetate/hexane = 1:30, v/v to give a yellowish brown oil (10.0 mg, 4% yield).

¹H NMR (400 MHz, CDCl₃) δ 10.77 (s, 1H), 7.63 - 7.52 (m, 6H), 7.16 - 7.11 (m, 2H), 6.75 (d, J = 10.1 Hz, 1H), 6.28 (s, 1H), 5.52 (d, J = 10.1 Hz, 1H), 5.12 - 5.08 (m, 1H), 2.82 (t, J = 8.0 Hz, 2H), 2.10 (q, J = 7.9 Hz, 2H), 1.74 - 1.66 (m, 9H), 1.58 (s, 4H), 1.41 (s, 3H), 1.40 - 1.35 (m, 4H), 0.92 - 0.87 (m, 3H).

### Example 24. 5-hydroxy-N-(4-hydroxyphenethyl)-2-methyl-2-(4-methylpent-3-en-1-yl)-7-pentyl-2H-chromene-6-carboxamide (4x)

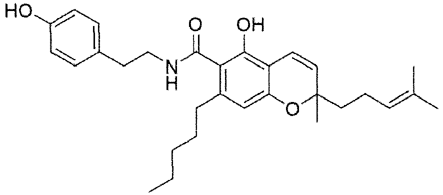

The compound was prepared according to Scheme 4 using cannabichromenic acid (CBCA) (3a) as starting material. It was purified by column chromatography on silica gel, with ethyl acetate/hexane = 1:30, v/v to give a yellowish brown oil (47.5 mg, 25% yield).

¹H NMR (400 MHz, CDCl₃) δ 11.51 (s, 1H), 7.11 - 7.08 (m, 2H), 6.80 - 6.77 (m, 2H), 6.72 (d, J = 10.1 Hz, 1H), 6.14 (s, 1H), 5.87 (s, 1H), 5.48 (d, J = 10.1 Hz, 1H), 5.10 - 5.06 (m, 1H), 4.72 (s, 1H), 3.71 (q, J = 6.4 Hz, 2H), 2.86 (t, J = 6.7 Hz, 2H), 2.48 (t, J = 8.0 Hz, 2H), 2.11 - 2.06 (m, 2H), 1.75 - 1.53(m, 12H), 1.47 - 1.40 (m, 3H), 1.37 (s, 3H), 1.22 - 1.14 (m, 5H), 0.87 (t, J = 7.1 Hz, 3H).

### Example 25. 5-hydroxy-N-(2-mercaptoethyl)-2-methyl-2-(4-methylpent-3-en-1-yl)-7-pentyl-2H-chromene-6-carboxamide (4y)

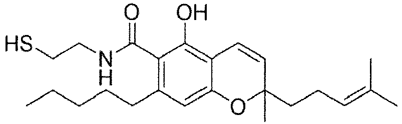

The compound was prepared according to Scheme 4 using cannabichromenic acid (CBCA) (3a) as starting material. It was purified by column chromatography on silica gel, with ethyl acetate/hexane = 1:20, v/v to give a yellow oil (33.8 mg, 20% yield).

¹H NMR (400 MHz, CDCl₃) δ 11.28 (s, 1H), 6.71 (d, J = 10.1 Hz, 1H), 6.35 (t, J = 5.8 Hz, 1H), 6.20 (s, 1H), 5.48 (d, J = 10.1 Hz, 1H), 5.10 - 5.06 (m, 1H), 3.81 (q, J = 6.0 Hz, 2H), 2.92 (t, J = 6.1 Hz, 2H), 2.72 (t, J = 8.0 Hz, 2H), 2.11 - 2.06 (m, 2H), 1.78 - 1.55 (m, 12H), 1.38 (s, 3H), 1.37 - 1.31 (m, 4H), 0.92 - 0.87 (m, 3H).

### Example 26. 5-hydroxy-2-methyl-2-(4-methylpent-3-en-1-yl)-7-pentyl-N-(piperidin-1-yl)-2H-chromene-6-carboxamide (4z)

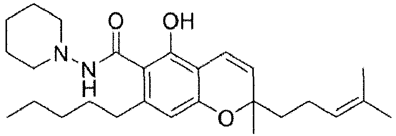

The compound was prepared according to Scheme 4 using cannabichromenic acid (CBCA) (3a) as starting material. It was purified by column chromatography on silica gel, with ethyl acetate/hexane = 1:20, v/v to give a clear oil (17.3 mg, 10% yield).

¹H NMR (400 MHz, CDCl₃) δ 6.71 (d, J = 10.1 Hz, 1H), 6.20 (s, 1H), 5.48 (d, J = 10.1 Hz, 1H), 5.12 - 5.05 (m, 1H), 2.84 (s, 3H), 2.69 (t, J = 8.0 Hz, 2H), 2.10 - 2.06 (m, 2H), 1.82 - 1.57 (s, 16H), 1.50 - 1.46 (m, 2H), 1.38 (s, 3H), 1.36 - 1.30 (m, 4H), 0.93 - 0.87 (m, 3H).

### Example 27. N-(2,3-dihydro-1H-inden-2-yl)-5-hydroxy-2-methyl-2-(4-methylpent-3-en-1-yl)-7-pentyl-2H-chromene-6-carboxamide (4aa)

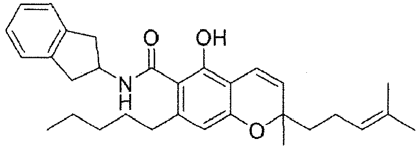

The compound was prepared according to Scheme 4 using cannabichromenic acid (CBCA) (3a) as starting material. It was purified by column chromatography on silica gel, with ethyl acetate/hexane = 1:20, v/v to give a yellow oil (86.1 mg, 45% yield).

¹H NMR (400 MHz, CDCl₃) δ 11.43 (s, 1H), 7.27 - 7.23 (m, 1H), 7.21 - 7.18 (m, 2H), 6.72 (d, J = 10.1 Hz, 1H), 6.13 - 6.11 (m, 2H), 5.48 (d, J = 10.1 Hz, 1H), 5.09 - 5.05 (m, 1H), 4.97 - 4.90 (m, 1H), 3.41 (d, J = 6.6 Hz, 1H), 3.37 (d, J = 6.6 Hz, 1H), 2.91 (d, J = 3.4 Hz, 1H), 2.87 (d, J = 3.4 Hz, 1H), 2.50 (t, J = 8.2 Hz, 2H), 2.11 - 2.05 (m, 2H), 1.76 - 1.59 (m, 7H), 1.56 (s, 3H), 1.47 - 1.40 (m, 2H), 1.37 (s, 3H), 0.85 (t, J = 7.2 Hz, 3H).

### Example 28. N-cycloheptyl-5-hydroxy-2-methyl-2-(4-methylpent-3-en-1-yl)-7-pentyl-2H-chromene-6-carboxamide (4ab)

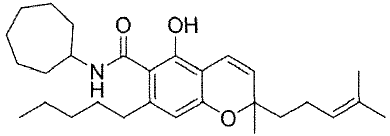

The compound was prepared according to Scheme 4 using cannabichromenic acid (CBCA) (3a) as starting material. It was purified by column chromatography on silica gel, with ethyl acetate/hexane = 1 : 30, v/v to give a clear oil (18.9 mg, 10% yield).

¹H NMR (400 MHz, CDCl₃) δ 11.33 (s, 1H), 6.72 (d, J = 10.1 Hz, 1H), 6.19 (s, 1H), 5.87 (d, J = 7.7 Hz, 1H), 5.48 (d, J = 10.1 Hz, 1H), 5.10 - 5.06 (m, 1H), 4.22 - 4.14 (m, 1H), 2.71 - 2.65 (m, 2H), 2.11 - 2.01 (m, 5H), 1.77 - 1.46 (m, 23H), 1.38 (s, 3H), 1.37 - 1.32 (m, 5H), 0.94 - 0.88 (m, 3H).

### Example 29. (3,4-dihydroisoquinolin-2(1H)-yl)(5-hydroxy-2-methyl-2-(4-methylpent-3-en-1-yl)-7-pentyl-2H-chromen-6-yl)methanone (4ac)

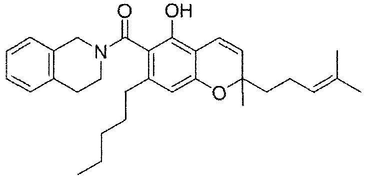

The compound was prepared according to Scheme 4 using cannabichromenic acid (CBCA) (3a) as starting material. It was purified by column chromatography on silica gel, with ethyl acetate/hexane = 1:20, v/v to give a yellow oil (31.2 mg, 17% yield).

¹H NMR (400 MHz, CDCl₃) δ 7.21 - 7.14 (m, 3H), 7.06 (s, 1H), 6.66 (d, J = 10.0 Hz, 1H), 6.28 (s, 1H), 5.52 - 5.49 (m, 1H), 5.13 - 5.08 (m, 1H), 4.66 - 4.60 (m, 1H), 3.84 - 3.76 (m, 1H), 2.92 - 2.86 (m, 2H), 2.44 (t, J = 7.9 Hz, 2H), 2.10 (q, J = 8.1 Hz, 2H), 1.78 - 1.58 (m, 10H), 1.52 - 1.47 (m, 2H), 1.39 (s, 3H), 1.25 - 1.13 (m, 4H), 0.83 - 0.78 (m, 3H).

### Example 30. N-(4-bromophenyl)-5-hydroxy-2-methyl-2-(4-methylpent-3-en-1-yl)-7-pentyl-2H-chromene-6-carboxamide (4ad)

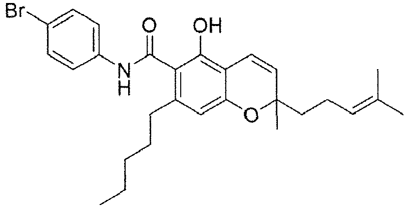

The compound was prepared according to Scheme 4 using cannabichromenic acid (CBCA) (3a) as starting material. It was purified by column chromatography on silica gel, with ethyl acetate/hexane = 1:30, v/v to give a yellowish brown oil (8 mg, 4% yield).

¹H NMR (400 MHz, CDCl₃) δ 10.65 (s, 1H), 7.52 (t, J = 2.5 Hz, 1H), 7.50 - 7.49 (m, 1H), 7.45 - 7.44 (m, 1H), 7.44 - 7.42 (m, 1H), 6.73 (d, J = 10.1 Hz, 1H), 6.27 (s, 1H), 5.52 (d, J = 10.1 Hz, 1H), 5.09 (t, J = 1.3 Hz, 1H), 2.78 (t, J = 8.0 Hz, 2H), 2.12 - 2.07 (m, 2H), 1.76 - 1.58 (m, 10H), 1.40 (s, 3H), 1.37 - 1.29 (m, 4H), 0.88 (t, J = 7.1 Hz, 3H).

### Example 31. (5-hydroxy-2-methyl-2-(4-methylpent-3-en-1-yl)-7-pentyl-2H-chromen-6-yl)(4-(2-hydroxyethyl)piperazin-1-yl)methanone (4ae)

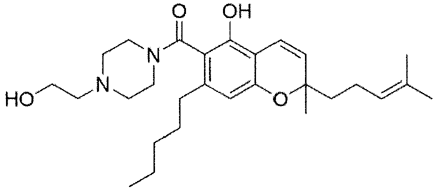

The compound was prepared according to Scheme 4 using cannabichromenic acid (CBCA) (3a) as starting material. It was purified by column chromatography on silica gel, with methanol/dichloromethane = 1 : 80, v/v to give a yellow oil (39.0 mg, 21% yield).

¹H NMR (400 MHz, CDCl₃) δ 6.65 (d, J = 10.1 Hz, 1H), 6.25 (s, 1H), 5.49 (d, J = 10.1 Hz, 1H), 5.12 - 5.07 (m, 1H), 3.79 (s, 1H), 3.66 (t, J = 5.2 Hz, 2H), 3.47 - 3.44 (m, 2H), 2.49 (t, J = 8.5 Hz, 2H), 2.43 (t, J = 7.9 Hz, 2H), 2.11 - 2.04 (m, 2H), 1.75 - 1.47 (m, 1 1H), 1.37 (s, 3H), 1.29 - 1.24 (m, 4H), 0.87 (t, J = 6.9 Hz, 3H).

### Example 32. 5-hydroxy-2-methyl-2-(4-methylpent-3-en-1-yl)-7-pentyl-N-(tetrahydro-2H-pyran-4-yl)-2H-chromene-6-carboxamide (4af)

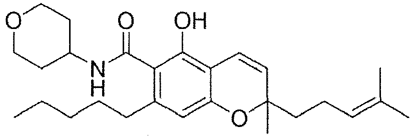

The compound was prepared according to Scheme 4 using cannabichromenic acid (CBCA) (3a) as starting material. It was purified by column chromatography on silica gel, with methanol/dichloromethane = 1 : 80, v/v to give a clear oil (41.5 mg, 19% yield).

¹H NMR (400 MHz, CDCl₃) δ 6.72 (d, J = 10.1 Hz, 1H), 6.20 (s, 1H), 5.83 (d, J = 7.4 Hz, 1H), 5.49 (d, J = 10.1 Hz, 1H), 5.10 - 5.05 (m, 1H), 4.27 - 4.19 (m, 1H), 4.02 - 3.98 (m, 2H), 3.58 - 3.50 (m, 2H), 2.71 - 2.65 (m, 2H), 2.13 - 2.00 (m, 5H), 1.78 - 1.49 (m, 16H), 1.38 (s, 3H), 1.37 - 1.33 (m, 5H), 0.94 - 0.88 (m, 3H).

### Example 33. N-cyclopentyl-5-hydroxy-N,2-dimethyl-2-(4-methylpent-3-en-1-yl)-7-pentyl-2H-chromene-6-carboxamide (4ag)

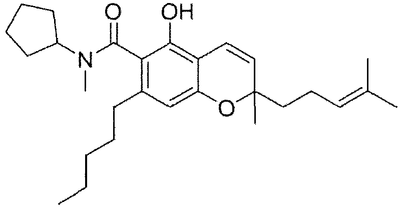

The compound was prepared according to Scheme 4 using cannabichromenic acid (CBCA) (3a) as starting material. It was purified by column chromatography on silica gel, with ethyl acetate/hexane = 1:20, v/v to give a yellow oil (49.7 mg, 28% yield).

¹H NMR (400 MHz, CDCl₃) δ 6.66 (d, J = 10.0 Hz, 1H), 6.27 (s, 1H), 5.51-5.48 (m, 1H), 5.12 - 5.07 (m, 1H), 4.55 (s, 1H), 2.88(s, 3H), 2.85 - 2.83(m, 1H), 2.48 - 2.33(m, 2H), 2.11 - 2.07(m, 2H), 1.84 - 1.82(m, 2H), 1.78 - 1.52(m, 22H), 1.40 - 1.36(m, 3H), 1.32 - 1.24(m, 7H), 0.87(t, J = 6.4 Hz, 3H).

### Experimental example 1. Evaluation of acetylcholinesterase and butyrylcholinesterase inhibitory efficacy of cannabichromenic acid derivative

Samples were prepared by dilution with 2.5% DMSO and 97.5% phosphate buffer to give concentrations of 0, 2, 5, 10, 20, 50, 100, and 200 µM of the synthesized compounds, and 50 µl was dispensed into a 96-well plate. Acetylcholinesterase or butyrylcholinesterase was prepared at 50 mU/ml, 50 µl was added to a 96-well plate, mixed with the aliquoted compound, and left for 10 minutes. The substrate, acetylthiocoline 0.05 mg/mL or butyrylthiocoline 0.1 mg/ml, was prepared and 50 µl was added to a 96-well plate and left for 10 minutes. Dithionitrobenzoic acid (DTNB) was prepared at 0.3 mg/ml, then 50 µl was added to a 96-well plate and left for 10 minutes. For an absorbance blank test, phosphate buffer was added instead of acetylthiocholine or butyrylthiocholine to correct for compound-induced absorbance values (positive control: rivastigmine).

### Experimental example 2. Evaluation of monoamine oxidase B (MAO-B) inhibitory efficacy of cannabichromenic acid derivative

Samples were prepared by dilution with DMSO to give concentrations of 0, 2, 5, 10, 20, 50, 100, and 200 µM of the compound, and 5 µl was dispensed into a 96-well plate. 135 µl of potassium phosphate buffer (0.1 M, 7.4 pH) was dispensed into a 96-well plate. Monoamine oxidase B was prepared at 2 mU/mL, and 50 µl was added to the 96-well plate, mixed with the dispensed compound, and left for 20 minutes. The substrate, kynuramine solution, was prepared at 100 µM, then 10 µl was added to a 96-well plate and left for 10 min. The reaction was stopped by adding 75 µl of NaOH (2N). The enzyme activity was calculated by measuring the concentration of kynuramine degraded by the enzyme using a fluorometer under 310 nm (excitation)/380 nm (emission) conditions. For the fluorescence blank experiment, potassium phosphate buffer was added instead of monoamine oxidase B to correct the compound-induced fluorescence intensity values.

The acetylcholinesterase (AChE) inhibitory efficacy, butyrylcholinesterase (BuChE) inhibitory efficacy, and monoamine oxidase B (MAOB) inhibitory efficacy measured as above are summarized in Table 1 below with IC₅₀ values for each compound.

**[Table 1]**

| compound | Inhibitory efficacy IC₅₀ (µM) | | |
|---|---|---|---|
| | IC₅₀ for AChE (µM) | IC₅₀ for BuChE (µM) | IC₅₀ for MAO-B (µM) |
| 4a | 1.3 | N.D. | N.D. |
| 4b | 0.4 | 94.6 | 176.0 |
| 4c | 4.3 | N.D. | N.D. |
| 4d | 1.0 | N.D. | N.D. |
| 4e | 0.4 | 0.4 | N.D. |
| 4f | 1.2 | N.D. | N.D. |
| 4g | 0.5 | N.D. | 110.8 |
| 4h | 2.3 | N.D. | N.D. |
| 4i | 0.3 | N.D. | 66.8 |
| 4j | 1.3 | N.D. | 43.3 |
| 4k | 1.3 | N.D. | 59.3 |
| 41 | N.D. | N.D. | 161.6 |
| 4m | 3.6 | N.D. | 79.7 |
| 4n | 1.2 | N.D. | 19.0 |
| 4o | N.D. | N.D. | 8.43 |
| 4p | 0.6 | N.D. | N.D. |
| 4q | N.D. | 6.2 | 131.4 |
| 4r | 1.1 | N.D. | 19.1 |
| 4s | 52.2 | N.D. | 15.5 |
| 4t | 47.7 | N.D. | 24..7 |
| 4u | N.D. | N.D. | N.D. |
| 4v | 6.6 | N.D. | N.D. |
| 4w | 13.7 | N.D. | 7.1 |
| 4x | 0.8 | 2.6 | N.D. |
| 4y | 6.2 | N.D. | N.D. |
| 4z | 29.0 | 1.3 | 156.3 |
| 4aa | 2.0 | 164.0 | 100.4 |
| 4ab | 2.6 | N.D. | N.D. |
| 4ac | 2.1 | N.D. | 137.1 |
| 4ad | <0.5 | N.D. | 12.0 |
| 4ae | <0.5 | 72.3 | 55.8 |
| 4af | 46.1 | N.D. | 194.1 |
| 4ag | 47.7 | N.D. | 14.4 |

| | | | |
|---|---|---|---|
| N.D.: Not Detected | | | |

As shown in Table 1 above, the synthesized cannabidiol derivatives were found to exhibit acetylcholinesterase and butyrylcholinesterase inhibitory activity as well as monoamine oxidase B inhibitory activity. In particular, a number of compounds exhibit butyrylcholinesterase inhibitory activity similar to that of the positive control rivastigmine [IC₅₀ values: acetylcholinesterase 1579.2 µM, butyrylcholinesterase 14.9 µM], and a number of compounds exhibit acetylcholinesterase inhibitory activity superior to that of rivastigmine, suggesting that they may be useful in the treatment of cognitive dysfunction, including dementia.

The foregoing description of the invention is for illustrative purposes only, and it will be readily apparent to those skilled in the art to which the invention belongs that varying substitutions and modifications may be made to the invention disclosed herein without departing from the spirit of the invention or essential features of the invention. It should therefore be understood that the embodiments described above are for the purpose of illustration of the invention only and are not intended in any way to limit the scope of the present invention. For example, each of the components described in a single form may also be implemented in a distributed manner, and similarly, components described as distributed may also be implemented in a combined form.

The scope of the invention is indicated by the following patent claims. The meaning and scope of the patent claims and all modifications or variations derived from their equivalents are considered to be falling within the scope of the invention.

## Claims

1. A cannabichromenic acid derivative compound represented by the following Formula 1, a hydrate thereof, a solvate thereof or a pharmaceutically acceptable salt thereof, wherein: 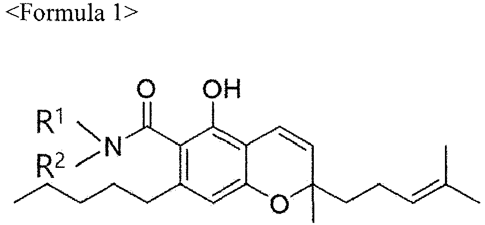
R¹ and R² are independently hydrogen; C₁₋₁₂ straight or branched alkyl chain; C₂₋₁₂ straight or branched alkenyl chain; C₂₋₅ straight or branched alkynyl chain; C₃₋₈ cycloalkyl; C₅₋₇ aryl; aryl C₁₋₄ alkyl; 5- to 7-membered heteroaryl having 1 to 3 heteroatom(s) selected from the group consisting of N and S; 5- to 7-membered heterocycloalkyl having 1 to 3 heteroatom(s) selected from the group consisting of N, O and S; or a fused bicyclic ring in which an aryl ring is fused with a non-aromatic cycloalkyl ring,
R¹ and R² are linked together to form a 5- to 7-membered non-aromatic heterocyclic ring having N linked to R¹ and R² and further having 0 to 2 heteroatoms selected from the group consisting of N and S,
the non-aromatic heterocyclic ring formed having R¹ and R² is fused with an aryl ring to form a fused bicyclic ring or not,
R¹ and R² are substituted with one or more R^{a} or not,
R^{a} is C₁₋₅ alkyl, halogen-substituted aryl, halogen-substituted heteroaryl, oxido, hydroxy, hydroxyalkyl, mercapto, alkylsulfonyl, halogen or butoxycarbonyl.

2. The cannabichromenic acid derivative compound, a hydrate thereof, a solvate thereof or a pharmaceutically acceptable salt thereof of Claim 1, **characterized in that** R¹ and R² is independently methyl, ethyl, isopropyl, butyl, nonyl, dodecyl, hexyl, undecenyl, octadienyl, propynyl, cyclopropyl, cyclopentyl, cyclohexyl, cycloheptyl, phenyl, pentyl, phenylpropyl, thiazolyl, tetrahydropyranyl, piperidinyl, or dihydroindenyl, or R¹ and R² are linked together to form thiomorpholino, piperazinyl, or dihydroisoquinolinyl.

3. The cannabichromenic acid derivative compound, a hydrate thereof, a solvate thereof or a pharmaceutically acceptable salt thereof of Claim 1, **characterized in that** R^{a} is methyl, ethyl, fluorophenyl, bromothiophenyl, oxido, hydroxy, hydroxyethyl, mercapto, methylsulfonyl, bromo or butoxycarbonyl.

4. The cannabichromenic acid derivative compound, a hydrate thereof, a solvate thereof or a pharmaceutically acceptable salt thereof of Claim 1, **characterized in that** the cannabichromenic acid derivative compound represented by Formula 1 is selected from the group consisting of:
[1] 5-hydroxy-*N*-isopropyl-2-methyl-2-(4-methylpent-3-en-1-yl)-7-pentyl-*2H-*chromen-6-carboxamide (4a),
[2] 5-hydroxy-2-methyl-2-(4-methylpent-3-en-1-yl)-7-pentyl-*N*-phenyl-*2H*-chromen-6- carboxamide (4b),
[3] *N*-cyclopropyl-5-hydroxy-2-methyl-2-(4-methylpent-3-en-1-yl)-7-pentyl-*2H-*chromen-6-carboxamide (4c),
[4] *N*-butyl-5-hydroxy-2-methyl-2-(4-methylpent-3-en-1-yl)-7-pentyl-*2H*-chromen-6-carboxamide (4d),
[5] *N*-cyclopentyl-5-hydroxy-2-methyl-2-(4-methylpent-3-en-1-yl)-7-pentyl-*2H-*chromen-6-carboxamide (4e),
[6] *N*-cyclohexyl-5-hydroxy-2-methyl-2-(4-methylpent-3-en-1-yl)-7-pentyl-*2H-*chromen-6-carboxamide (4f),
[7] 5-hydroxy-2-methyl-2-(4-methylpent-3-en-1-yl)-*N*-nonyl-7-pentyl-*2H*-chromen-6-carboxamide (4g),
[8] *N*-dodecyl-5-hydroxy-2-methyl-2-(4-methylpent-3-en-1-yl)-7-pentyl-*2H-*chromen-6-carboxamide (4h),
[9] 5-hydroxy-2-methyl-2-(4-methylpent-3-en-1-yl)-7-pentyl-*N*-(undec-10-en-1-yl)-2H-chromen-6-carboxamide (4i),
[10] *N*-(2-ethylhexyl)-5-hydroxy-2-methyl-2-(4-methylpent-3-en-1-yl)-7-pentyl-*2H-*chromen-6-carboxamide (4j),
[11] (E)-*N*-(3,7-dimethylocta-2,6-dien-1-yl)-5-hydroxy-2-methyl-2-(4-methylpent-3-en-1-yl)-7-pentyl-*2H*-chromen-6-carboxamide (4k),
[12] (1,1 -dioxidothiomorpholino)(5-hydroxy-2-methyl-2-(4-methylpent-3-en-1-yl)-7-pentyl-*2H*-chromen-6-yl)methanone (41),
[13] 5-hydroxy-2-methyl-2-(4-methylpent-3-en-1-yl)-7-pentyl-*N*-(prop-2-yn-1-yl)-*2H*-chromen-6-carboxamide (4m),
[14] 5-hydroxy-2-methyl-2-(4-methylpent-3-en-1-yl)-7-pentyl-*N*-(3-phenylpropyl)-*2H*-chromen-6-carboxamide (4n),
[15] 5-hydroxy-2-methyl-2-(4-methylpent-3-en-1-yl)-*N*-(1-(methylsulfonyl)piperidin-4-yl)-7-pentyl-*2H*-chromen-6-carboxamide (4o),
[16] *N*-((4-bromothiophen-2-yl)methyl)-5-hydroxy-2-methyl-2-(4-methylpent-3-en-1-yl)-7-pentyl-*2H*-chromen-6-carboxamide (4p),
[17] 5-hydroxy-2-methyl-2-(4-methylpent-3-en-1-yl)-*N*-(1-(methylpiperidin-4-yl)-7-pentyl-*2H*-chromen-6-carboxamide (4q),
[18] 5-hydroxy-2-methyl-2-(4-methylpent-3-en-1-yl)-*N*-(5-methylthiazol-2-yl)-7-pentyl-*2H*-chromen-6-carboxamide (4r),
[19] *tert*-butyl(3S)-3-(5-hydroxy-2-methyl-2-(4-methylpent-3-en-1-yl)-7-pentyl-*2H-*chromen-6-carboxamido)piperidin-1-carboxylate (4s),
[20] 5-hydroxy-2-methyl-2-(4-methylpent-3-en-1-yl)-7-pentyl-*N*-(p-tolyl)-*2H-*chromen-6-carboxamide (4t),
[21] *tert*-butyl 4-(5-hydroxy-2-methyl-2-(4-methylpent-3-en-1-yl)-7-pentyl-*2H-*chromen-6-carboxamido)piperidin-1-carboxylate (4u),
[22] *N*-(*sec*-butyl)-5-hydroxy-2-methyl-2-(4-methylpent-3-en-1-yl)-7-pentyl-*2H-*chromen-6-carboxamide (4v),
[23] *N*-(4'-fluoro-[1,1'-biphenyl]-4-yl)-5-hydroxy-2-methyl-2-(4-methylpent-3-en-1-yl)-7-pentyl-*2H*-chromen-6-carboxamide (4w),
[24] 5-hydroxy-*N*-(4-hydroxyphenethyl)-2-methyl-2-(4-methylpent-3-en-1-yl)-7-pentyl-*2H*-chromen-6-carboxamide (4x),
[25] 5-hydroxy-*N*-(2-mercaptoethyl)-2-methyl-2-(4-methylpent-3-en-1-yl)-7-pentyl-2H-chromen-6-carboxamide (4y),
[26] 5-hydroxy-2-methyl-2-(4-methylpent-3-en-1-yl)-7-pentyl-*N*-(piperidin-1-yl)-*2H-*chromen-6-carboxamide (4z),
[27] *N*-(2,3-dihydro-1H-inden-2-yl)-5-hydroxy-2-methyl-2-(4-methylpent-3-en-1-yl)-7-pentyl-*2H*-chromen-6-carboxamide (4aa),
[28] *N*-cycloheptyl-5-hydroxy-2-methyl-2-(4-methylpent-3-en-1-yl)-7-pentyl-*2H-*chromen-6-carboxamide (4ab),
[29] (3,4-dihydroisoquinolin-2(1H)-yl)(5-hydroxy-2-methyl-2-(4-methylpent-3-en-1- yl)-7-pentyl-*2H*-chromen-6-yl)methanone (4ac),
[30] *N*-(4-bromophenyl)-5-hydroxy-2-methyl-2-(4-methylpent-3-en-1-yl)-7-pentyl-*2H*-chromen-6-carboxamide (4ad),
[31] (5-hydroxy-2-methyl-2-(4-methylpent-3-en-1-yl)-7-pentyl-*2H*-chromen-6-yl)(4-(2-hydroxyethyl)piperazin-1-yl)methanone (4ae),
[32] 5-hydroxy-2-methyl-2-(4-methylpent-3-en-1-yl)-7-pentyl-*N*-(tetrahydro-*2H-*pyran-4-yl)-2H-chromen-6-carboxamide (4af),
[33] *N*-cyclopentyl-5-hydroxy-N,2-dimethyl-2-(4-methylpent-3-en-1-yl)-7-pentyl-*2H*-chromen-6-carboxamide (4ag).

5. A pharmaceutical composition for cognitive function improvement, comprising the cannabichromenic acid derivative compound, a hydrate thereof, a solvate thereof or a pharmaceutically acceptable salt thereof of any one of Claims 1 to 4 as an active ingredient.

6. The pharmaceutical composition of Claim 5, **characterized in that** the cognitive function improvement is preventing or treating degenerative brain disease.

7. The pharmaceutical composition of Claim 6, **characterized in that** the degenerative brain disease is one or more selected from the group consisting of Alzheimer's disease, mild cognitive impairment, stroke and vascular dementia, frontotemporal dementia, Lewy body dementia, Creutzfeldt-Jakob disease, traumatic head injury, syphilis, acquired immunodeficiency syndrome and other viral infections, brain abscess, brain tumor, multiple sclerosis, dementia caused by metabolic diseases, hypoxia, Parkinson's disease, Lou Gehrig's disease, Huntington's disease, Pick's disease, amyotrophic lateral sclerosis, epilepsy, ischemia, stroke, attention deficit hyperactivity disorder, schizophrenia, depression, bipolar disorder, post-traumatic stress disorder, spinal cord injury, and myelitis.

8. A composition for inhibiting monoamine oxidase (MAO) comprising the cannabichromenic acid derivative compound, a hydrate thereof, a solvate thereof or a pharmaceutically acceptable salt thereof of any one of Claims 1 to 4, as an active ingredient.

9. A food composition for cognitive function improvement comprising the cannabichromenic acid derivative compound, a hydrate thereof, a solvate thereof or a pharmaceutically acceptable salt thereof of any one of Claims 1 to 4, as an active ingredient.

10. Method for preparing a cannabichromenic acid derivative compound represented by the following Formula 1, comprising:
reacting (E)-non-3-en-2-one represented by Formula 1-1 below with dimethyl malonate represented by Formula 1-2 below in an organic solvent to obtain a first compound represented by Formula 1a below;
mixing the first compound obtained above with citral and calcium hydroxide, reacting in an organic solvent to obtain a second compound represented by Formula 2a below;
reacting the second compound obtained above with thiophenol and cesium carbonate to obtain a third compound represented by Formula 3a; and
reacting the third compound obtained above with 1-hydroxybenzotriazole, 1-(3-dimethylaminopropy)-3-ethylcarbodiimide hydrochloride and an amine compound, followed by purification to obtain a cannabichromenic acid derivative compound represented by Formula 1 below: 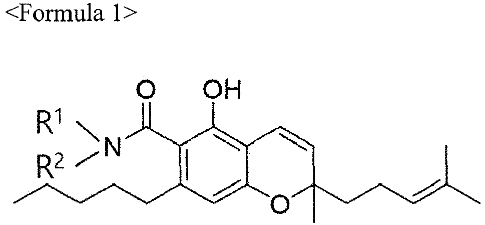 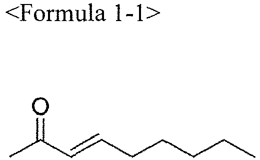 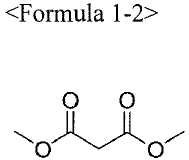 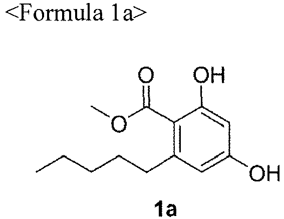 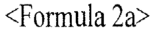 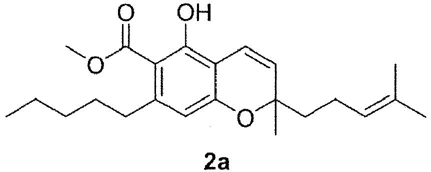 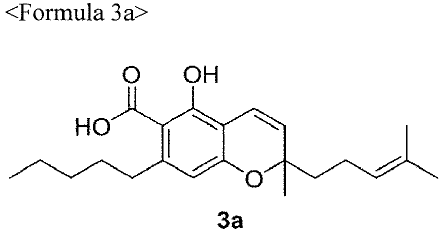
wherein in Formula 1, R¹ and R² are the same as defined in Claim 1.
